# EUROPEAN PATENT SPECIFICATION

(11) **EP 2 288 358 B1**
(45) Date of publication and mention of the grant of the patent: **23.09.2015**
(21) Application number: 09732459.4
(22) Date of filing: 16.04.2009
(51) Int. Cl.: C07K 1/30, A61K 35/12

(54) **METHOD OF ISOLATING ANTIBODIES USING POLYALKYLENE GLYCOL AND TRANSITION METALS**
VERFAHREN ZUR ISOLIERUNG VON ANTIKÖRPERN MIT POLYALKYLENGLYKOL UND ÜBERGANGSMETALLEN
PROCÉDÉ D'ISOLEMENT D'ANTICORPS À L'AIDE DE POLYALKYLÈNE GLYCOL ET DE MÉTAUX DE TRANSITION

(30) Priority: 16.04.2008 US 71182 P
(43) Date of publication of application: 02.03.2011
(73) Proprietor: Biogen MA Inc., Cambridge, MA 02142 (US)
(72) Inventor: GRONKE, Robert, S., Boston MA 02116 (US); JAQUEZ, Orlando, A., Revere MA 02151 (US)
(74) Representative: Adams, Harvey Vaughan John
(86) International application number: PCT/US2009/002390
(87) International publication number: WO 2009/128935

(56) References cited:
- EP-A1- 1 044 690
- WO-A1-93/14110
- WO-A1-2008/068455
- US-A- 5 135 875
- US-A- 5 907 035
- US-A1- 2005 271 654
- US-B1- 6 391 609
- WICKERHAUSER M ET AL: "Large scale preparation of macroglobulins", VOX SANGUINIS, S. KARGER AG, BASEL, CH, vol. 23, no. 1, 1 July 1972 (1972-07-01), pages 119-125, XP009097174, ISSN: 0042-9007
- ATHA D H ET AL: "MECHANISM OF PRECIPITATION OF PROTEINS BY POLY ETHYLENE GLYCOLS ANALYSIS IN TERMS OF EXCLUDED VOLUME", JOURNAL OF BIOLOGICAL CHEMISTRY.(MICROFILMS), AMERICAN SOCIETY OF BIOLOGICAL CHEMISTS, BALTIMORE, MD, US, vol. 256, no. 23, 1 January 1981 (1981-01-01), pages 12108-12117, XP002454548,
- THRASH S L ET AL: "Effect of divalent ions on protein precipitation with polyethylene glycol: Mechanism of action and applications", PROTEIN EXPRESSION AND PURIFICATION, ACADEMIC PRESS, SAN DIEGO, CA, vol. 2, no. 1, 1 February 1991 (1991-02-01), pages 83-89, XP024800053, ISSN: 1046-5928, DOI: 10.1016/1046-5928(91)90015-B [retrieved on 1991-02-01]
- 'JB Screen PEG/Salt2. Datasheet' JENA BIOSCIENCE, [Online] 11 July 2007, XP008161525 Retrieved from the Internet: <URL:<http://web.archive.org/web/2007071114 0106/http://www.mitigen.com/mic_catalog.php c?c=jenS creenPEGSalt>> [retrieved on 2009-06-01]

## Description

### BACKGROUND OF THE INVENTION

The present invention is related to a method of isolating a biological macromolecule in a composition. Specifically, the present invention is related to a method of isolating an antibody in a composition containing an impurity, the method comprising adding a polyalkylene glycol to the composition, adding a transition metal to the composition, and separating the biomacromolecule from the impurity.

Biological macromolecules (*i.e*., biomacromolecules), such as recombinant proteins or antibodies, have importance in a diverse array of technologies. Traditionally, biomacromolecules have been purified using several different methods, *e.g.,* filtration, centrifugation, size exclusion chromatography, affinity chromatography, ion exchange chromatography, immobilized metal affinity chromatography, or combinations of the above, just to name a few. The method of purification is generally chosen based on a characteristic of the biomacromolecule that distinguishes it from one or more impurities that coexist with the biomacromolecule in a composition, e.g., size, charge, or affinity for a ligand. A vast number of biomacromolecules are commercially important, and an ability to purify a large amount of biomacromolecules in a timely and cost effective manner is desired.

Commercially important biomacromolecules include, *e.g.*, proteins and nucleic acids, *e.g.,* DNA and RNA. Two examples of biomacromolecules that are often isolated on an industrial scale are monoclonal antibodies and fusion proteins. These antibodies and fusion proteins are valuable in various diagnostic and therapeutic fields, and have been used to treat various diseases such as inherited and acquired immune-deficiency diseases and infectious diseases.

The harvesting of biomacromolecules from industrial-scale bioreactors containing mammalian or bacterial cells is generally performed using either filtration or centrifugation. However, the recent drive for generating increased amounts of protein production in cell culture has required bioreactors to operate at higher cell densities, which increases the amount of impurities such as DNA, host cell proteins, and other media components. The elevated levels of contaminants have placed stronger demands on both cell harvesting operations (e.g., the filtration and centrifugations steps), as well as the downstream purification steps (e.g., chromatography and dialysis steps). The presence of these higher concentrations of impurities may increase the number of purification steps that need to be performed, thus increasing cost and decreasing overall production throughput. Increased protein production can even saturate the capacity of some chromatographic methods.

Traditional approaches to producing purified antibodies can include centrifugation, ion exchange chromatography *(e.g.,* DEAE or hydroxyapatite), immunoaffinity purification (*e.g.,* protein A or protein G), and dialysis. See *e.g.,* Antibodies: A Laboratory Manual, Harlow and Lane, Cold Spring Harbor Laboratory (1988). The use of a combination of the above methods is common, *e.g.,* antibody purification from plasma using ethanol fractionation followed by ion exchange chromatography and/or caprylic acid (CA) precipitation. See for example McKinney et al., J. Immunol. Methods 96:271-278 (1987); U.S. Pat. Nos. 4,164,495; 4,177,188; RE 31,268; 4,939,176; 5,164,487 and WO 2008/100578. In addition, acidification of fermentation has been used to improve recovery and stability of antibodies and recombinant proteins. See *e.g.,* Lydersen et al., Annals New York Academy of Sciences 745:222-31 (1994).

Various other methods have been developed for isolation and/or purification of antibodies. See *e.g.,* U.S. Pat. Nos. 7,038,017; 7,064,191; 6,846,410; 5,429,746; 5,151,504; 5,110,913; 4,933,435; 4,841,024; and 4,801,687. However, many of these methods can result in large feedstock volumes and recovery loss, have high production costs on an industrial scale, and/or have low throughputs.

Precipitation of biomacromolecules induced by the introduction of a precipitating agent can be a quick and effective method to recover biomacromolecules. Traditional precipitation techniques include ammonium sulfate precipitation and caprylic acid precipitation. One traditional drawback to precipitation is that it requires the removal of the precipitating agents after the precipitation is complete. Due to this drawback, precipitation is often used as an upstream purification method. Another drawback of precipitation is the requirement of large concentration of the precipitating agent to achieve the desired result, thus creating large amounts of waste product.

Polyethylene glycol (PEG) has been used previously to precipitate various biomacromolecules. See, e.g, Ingham, K., "Precipitations of Proteins with Polyethylene Glycol," 301-306 (1990) and WO 2008/100578. PEG was found to be nondenaturing in various environments. However, high concentrations of PEG are generally used to achieve the desired result, e.g., 20% of the total volume. The high concentrations of PEG result in a significant increase in volume, create a large amount of waste, and increase the viscosity of the composition. Additionally, the required high concentrations of PEG increase the costs associated with purification.

Metal affinity precipitation has also previously been attempted. See, e.g., Van Dam, M. et al., "Metal Affinity Precipitation of Proteins," Biotechno. Appl. Biochem, 492-502 (1989) and Zaworski, P.G., et al., "Precipitation of Proteins from Culture Supernatants Using Zinc," Analytical Biochem 440-444 (1988). However, high concentrations of zinc are also required to achieve the desired precipitation (up to 80 mM). Thus, the use of metal affinity at these high concentrations produces significant amounts of waste and significantly increases cost.

As a result of the aforementioned difficulties and inefficiencies, there is a need to improve the strategy for isolation of biomacromolecules.

### SUMMARY OF THE INVENTION

The present invention provides a method of isolating an antibody in a composition containing an impurity, the method comprising: (a) adding a polyalkylene glycol to the composition; (b) adding a transition metal to the composition; (c) allowing said antibody to form a precipitate in the presence of said polyalkylene glycol and transition metal; and (d) separating said precipitated antibody from said impurity, wherein step (a) and step (b) can be carried out in any order.

Various polyalkylene glycols can be used. In some embodiments, the polyalkylene glycol is selected from the group consisting of polypentylene glycol, polybutylene glycol, polypropylene glycol, or polyethylene glycol. In some embodiments, the polyalkylene glycol is a polyethylene glycol. Various molecular weights of polyalkylene glycols can be used. In some embodiments, the polyethylene glycol has a molecular weight of between 1,000 Da and 20,000 Da. In some embodiments, the polyethylene glycol has a molecular weight of between 2,000 Da and 15,000 Da.

Various concentrations of polyalkylene glycols can be used in the method of the present invention. In some embodiments, the concentration of the polyalkylene glycol in the composition is about 0.5% to about 30% (w/v). In some embodiments, the polyalkylene glycol in the composition is about 2.0% to about 10%(w/v). In some embodiments of the present invention, the polyalkylene glycol is in the composition at about 1%-2% (w/v).

Transition metals can be used in the method of the present invention to increase separation of the antibody from an impurity. In some embodiments, the transition metal is selected from the group consisting of zinc, nickel, copper, cobalt or manganese. In some embodiments, the transition metal is zinc. Various concentrations of transition metals can be used. In some embodiments, the concentration of the transition metal in the composition is about 1 mM to about 50 mM. In some embodiments, the concentration of the transition metal in the composition is about 2.5 mM to about 15 mM. In some embodiments, the concentration of the transition metal in the composition is about 0.5 mM to about 15 mM. In some embodiments, the concentration of the transition metal in the composition is about 1.5 mM-3.5mM.

Various methods can be used to separate the ant ibody from an impurity. In some embodiments, the separation is performed by filtering the composition, the filtering forming a permeate stream and a solid stream. In some embodiments, the filtering is performed by a dead-end filter. In some embodiments, the antibody is substantially in the solid stream. In some embodiments, the separating is performed by filtering the composition, the antibody remaining in the solid, and wherein the filtering results in a transmembrane pressure; and wherein the transmembrane pressure remains substantially constant during the filtering. In some embodiments, the separating is performed by subjecting the composition to centrifugation, the centrifugation forming a supernatant and a pellet In some embodiments, the ant ibo dy is substantially in the pellet.

In some embodiments, the present invention is a method to isolate an antibody from a composition containing an impurity. In some embodiments, the composition comprises eukaryotic cellular material. In some embodiments, the impurity is selected from the group consisting of a growth media components, protein, lipid, nucleic acid, ribonucleic acid, and combinations thereof.

In some embodiments, the method of present invention increases recovery of an antibody from a composition, relative to methods which only utilize a polyalkylene glycol or a transition metal. In some embodiments, the addition of the polyalkylene glycol and transition metal increases the recovery of the ant ibody by greater than 3%. In some embodiments, the recovery of the polyalkylene glycol and transition metal increases the recovery of the antibody by greater than 10%. In some embodiments the recovery of the antibody is synergistic when using both a polyalkylene glycol and a transition metal, as compared to use of a polyalkylene glycol or a transition metal alone.

In some embodiments of the present invention, the polyalkylene glycol is a polyethylene glycol having a molecular weight of between 1,000 Da and 20,000 Da, the transition metal is zinc, the separating is performed by precipitating the antibody, in the solution and then centrifuging the composition to isolate the precipitate.

In some embodiments of the present invention, the polyalkylene glycol is a polyethylene glycol having a molecular weight of between 1,000 Da and 20,000 Da, the transition metal is zinc, the separating is performed by precipitating the antibody in the solution and then filtering the composition to isolate the antibody.

The present invention and embodiments thereof are set out in the appended claims.

### BRIEF DESCRIPTION OF THE DRAWINGS/FIGURES

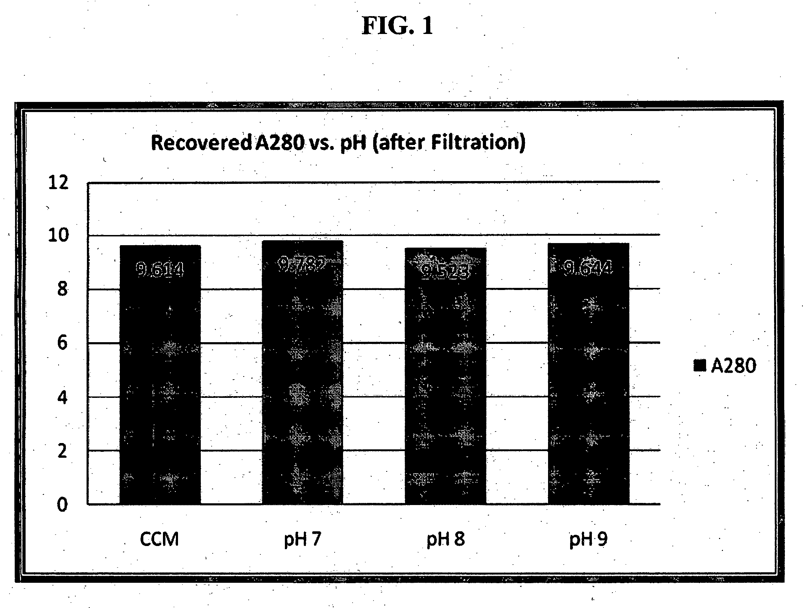

FIG. 1 depicts the effect of pH on Ab C precipitation. Samples of Ab C were titrated to pH 7, pH 8, and pH 9 and were held for 1hour (isoelectric point of Ab C is ∼8.5). Samples were then filtered to capture precipitate (if any) and absorbance at A₂₈₀ were taken. Absorbance measurements for titrated samples were compared to the starting material ("CCM"). Recovered absorbance for samples titrated to pH 7, pH 8, and pH 9 were 101.7%, 99.1%, 100.3%, respectively. This indicates that pH alone is not sufficient to induce precipitation at the harvested product concentration.

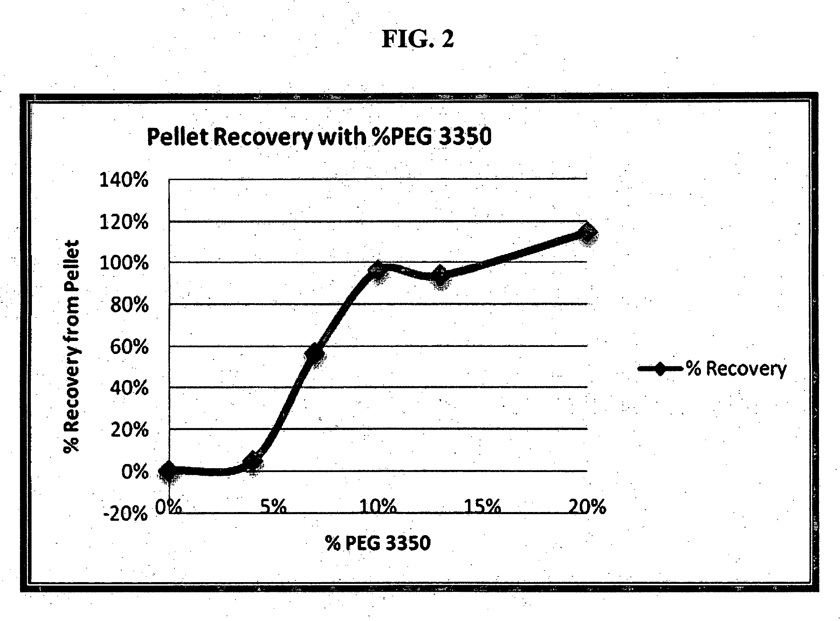

FIG. 2 depicts the effect of PEG 3350 concentration (w/v) on precipitation and recovery of AbC. Samples of harvested Ab C were adjusted to pH 9.0 and were treated with 0% PEG 3350, 4% PEG 3350, 7% PEG 3350, 10% PEG 3350, 13% PEG 3350, and 20% PEG 3350. Samples were held for 30 - 60 min at 2-8°C and were centrifuged at 14,000 rpm for 3 min. Supernatants were decanted and pellets were resuspended in resuspension buffer. Recovery was calculated by size-exclusion chromatography, using the harvested Ab C sample as a control. At least 10% PEG 3350 was required to precipitate Ab C and achieve recoveries of >95%.

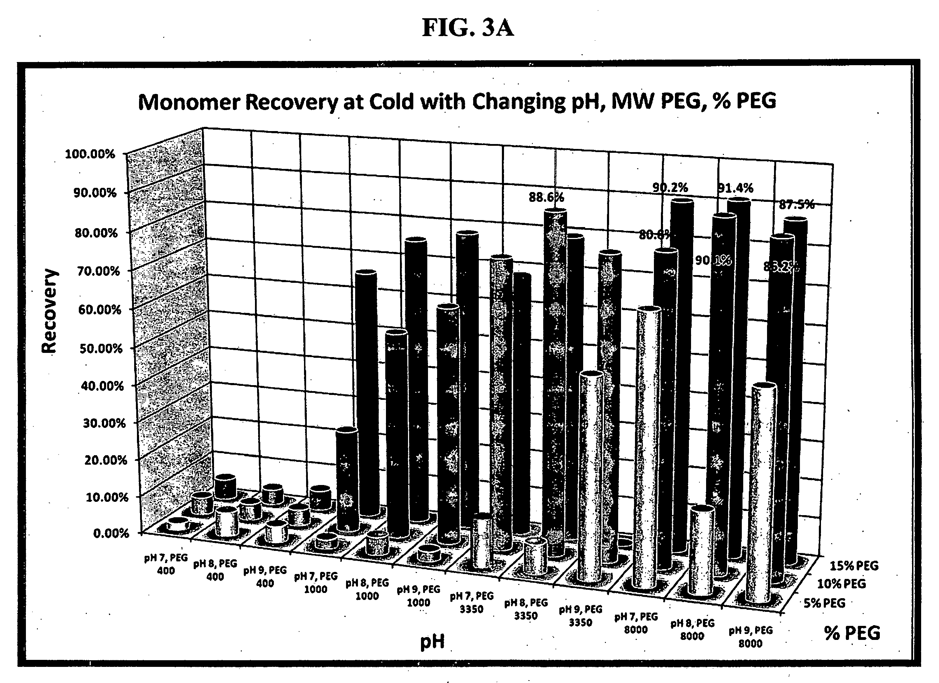

FIG. 3A depicts the effects of pH and molecular weight of PEG at varying PEG concentrations. Samples of Ab C were titrated to pH 7,8, and 9 and were treated with 5%, 10%, or 15% of PEG molecular weight 400, 1000, 3350, and 8000 Da. Data shows that PEG 1000-PEG 8000 all produce high recoveries at amounts ≥ 5%. Samples were held for 30 - 60 min at 2-8°C and were centrifuged at 14,000 rpm for 3 min. Supernatants were decanted and pellets were resuspended in a resuspension buffer. Recovery was calculated by size-exclusion chromatography, using the harvested Ab C sample as a control.

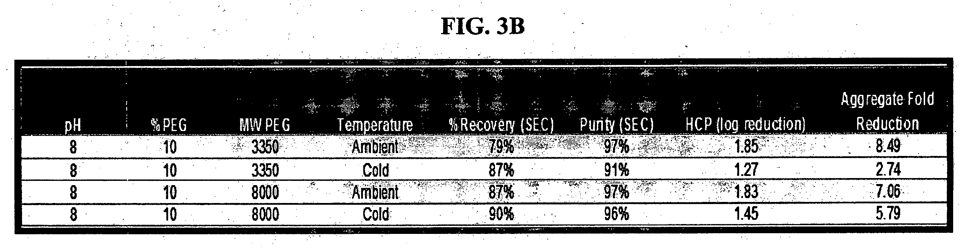

FIG. 3B depicts the effect of PEG molecular weight on Ab C recovery. Samples of Ab C were titrated to pH 8 and were treated with 10% PEG 3350 or PEG 8000 at either 2-8°C or ambient temperatures. Samples were held for 30 - 60 min at 2-8°C or at ambient temperature, and were centrifuged at 14,000 rpm for 3 min. Supernatants were decanted and pellets were resuspended in a resuspension buffer. Recovery was calculated by size-exclusion chromatography, using the harvested Ab C sample as a control. Both, PEG 3350 and PEG 8000 result in high yield.

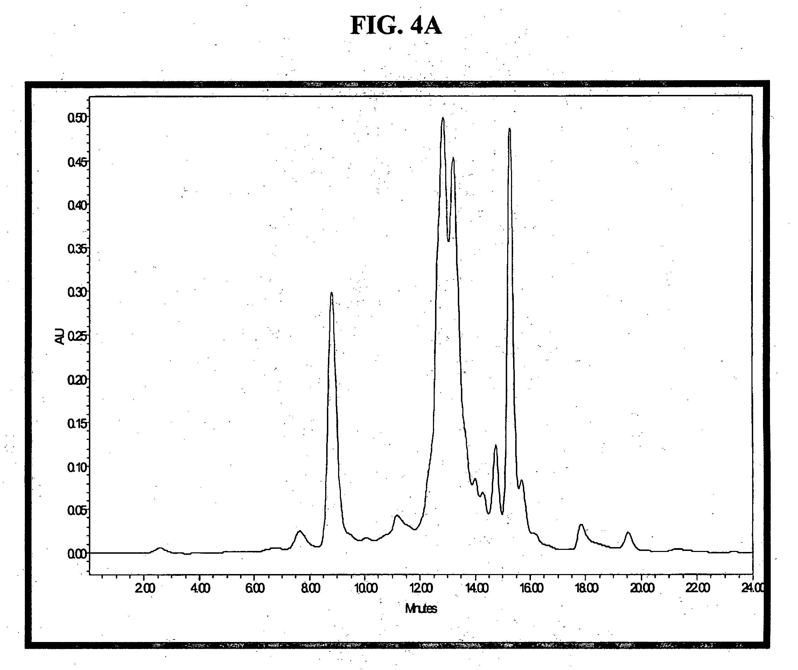

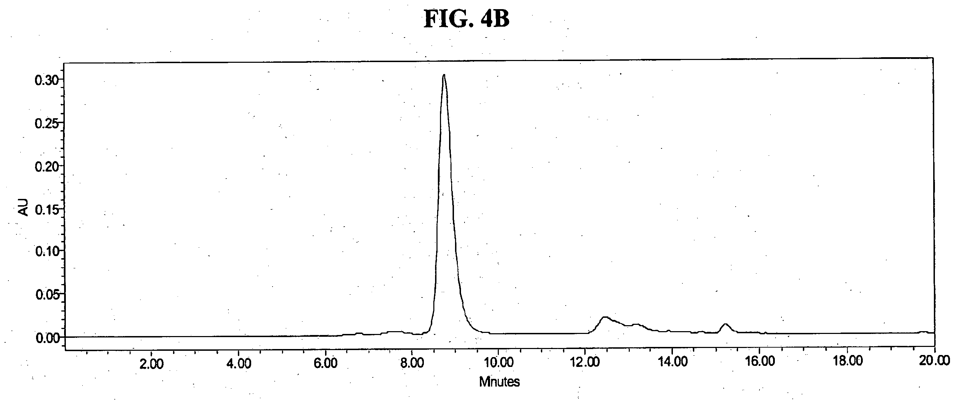

FIG. 4A depicts the purity of an antibody Ab C formulation before PEG precipitation, and FIG 4B represents the purity of the antibody Ab C formulation after being precipitated with 10% PEG 8000 at pH 8.0.

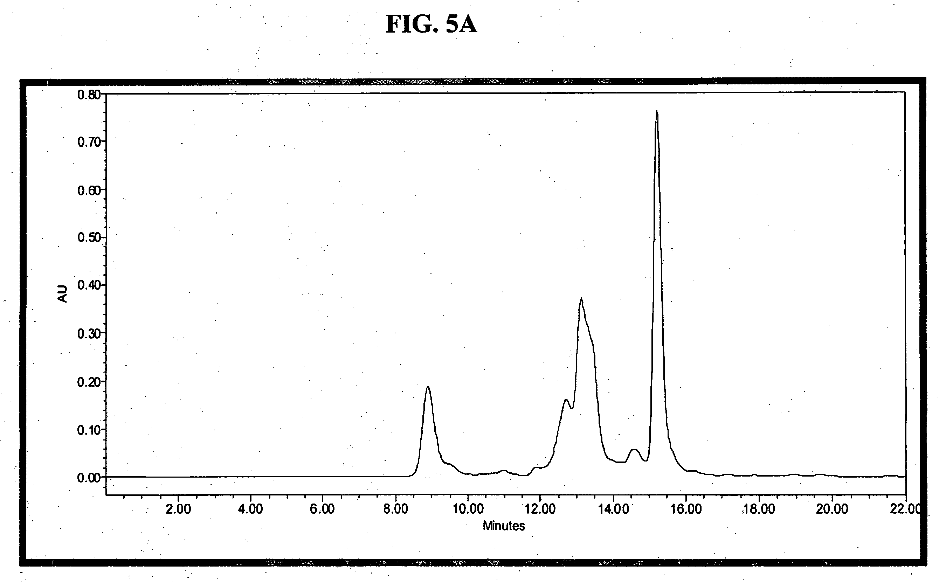

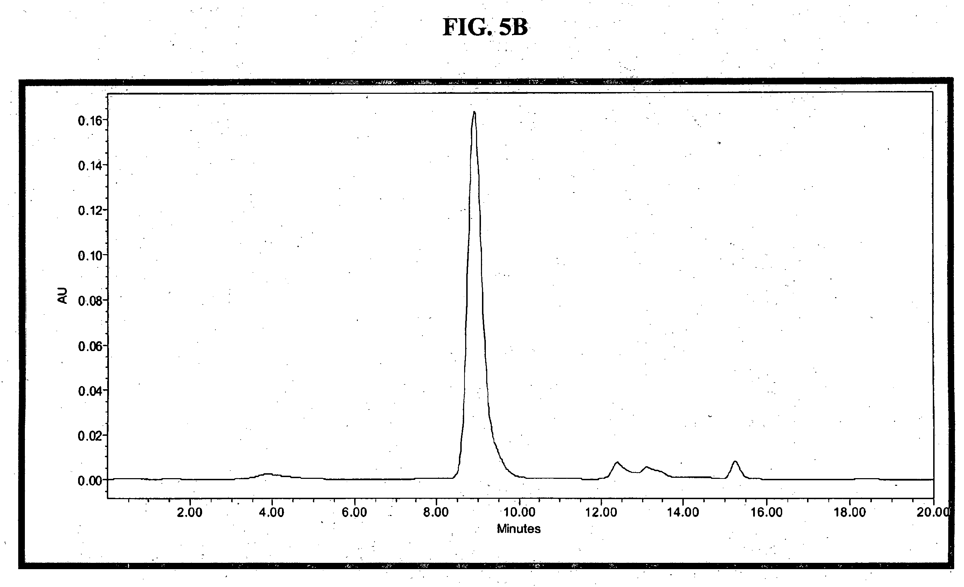

FIG. 5A depicts the purity of an antibody Ab D formulation before PEG precipitation, and FIG. 5B represents the purity of the antibody Ab D formulation after being precipitated with 10% PEG 8000 at pH 8.0.

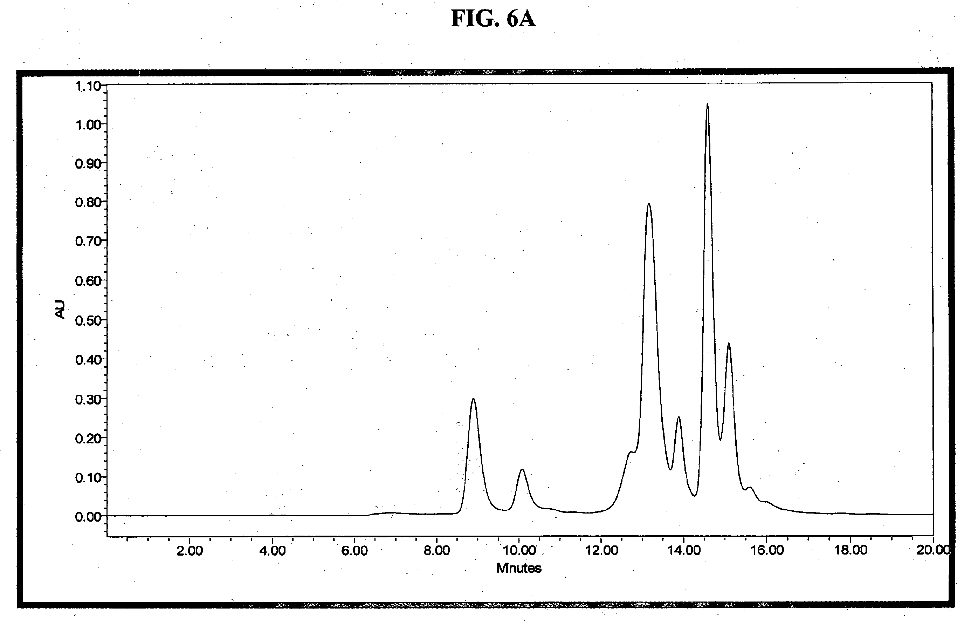

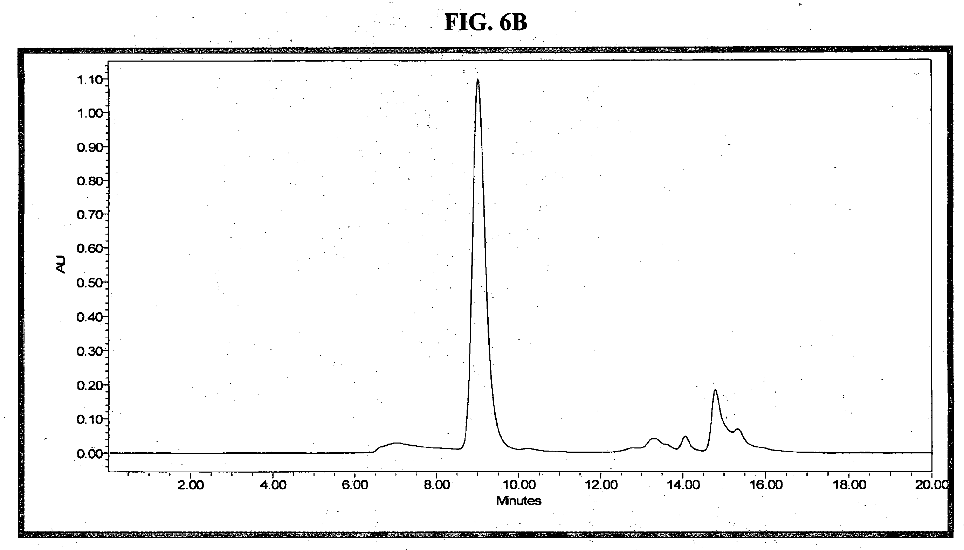

FIG. 6A depicts the purity of an antibody Ab E formulation before PEG precipitation, and FIG. 6B represents the purity of the antibody Ab E formulation after being precipitated with 10% PEG 8000 at pH 8.0.

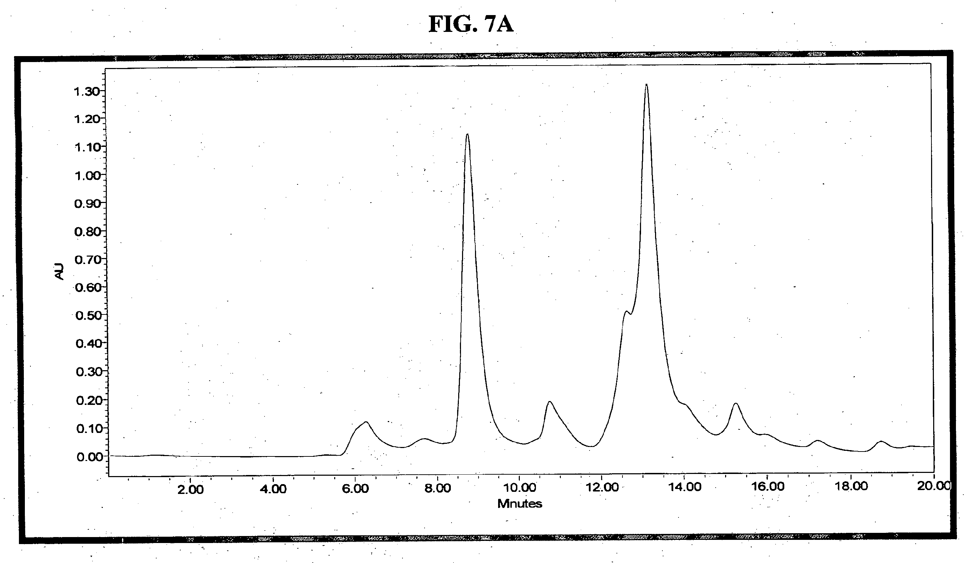

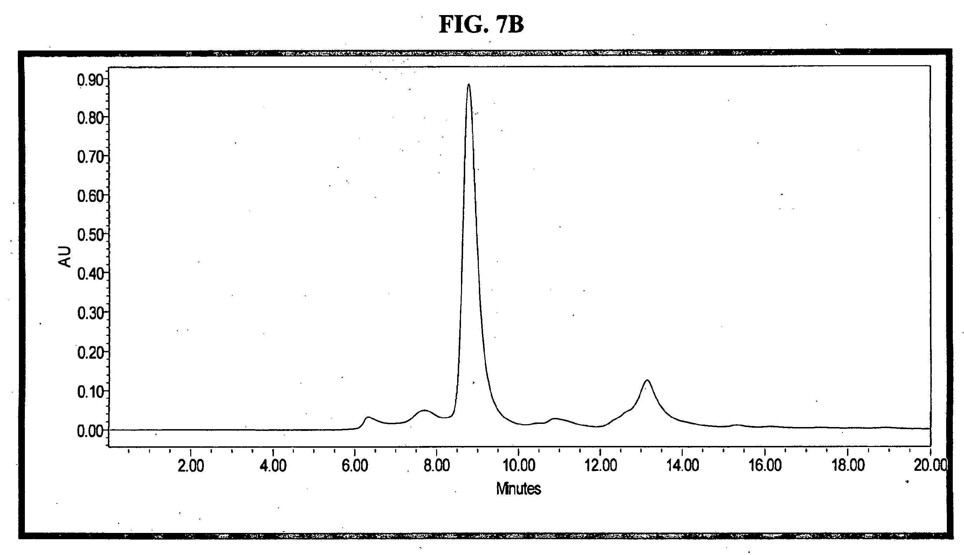

FIG. 7A depicts the purity of an antibody Ab F formulation before PEG precipitation, and FIG. 7B represents the purity of the antibody Ab F formulation after being precipitated with 10% PEG 8000 at pH 8.0.

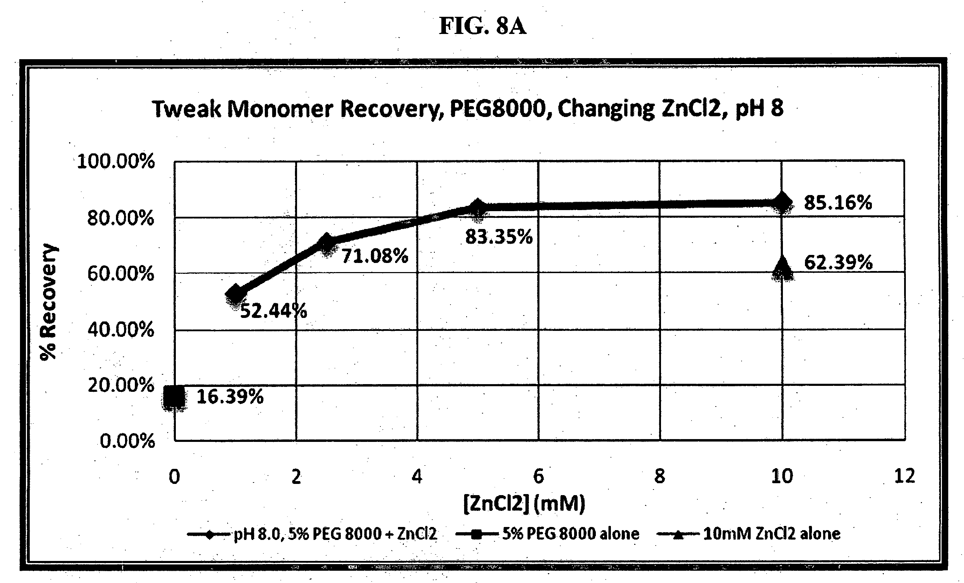

FIG. 8A depicts the effect of 0 mM ZnCl₂ and 10 mM ZnCl₂ on precipitation of Ab C. Also presented is the effect of adding 5% PEG at various ZnCl₂ concentrations.

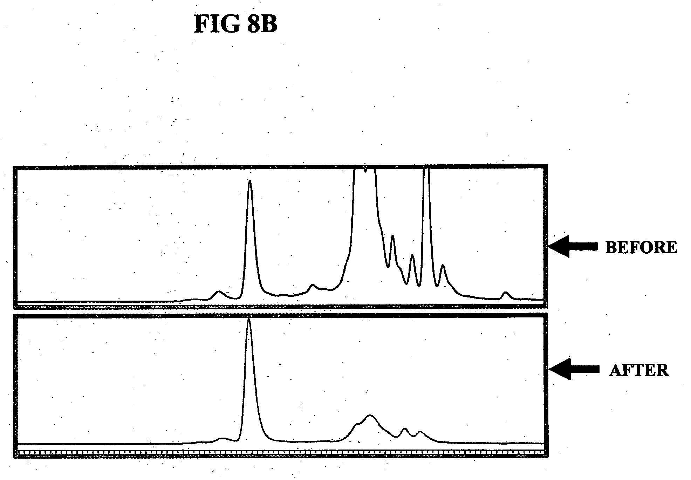

FIG. 8B depicts the purity of an antibody Ab C formulation before PEG precipitation, and after being precipitated with 5% PEG 3350 and 5mM ZnCl₂ at pH 7.2
FIG. 9 depicts the effect that various PEG and ZnCl₂ concentrations have on the precipitation of the an antibody in a composition at pH 7.0.
FIG. 10 depicts the effect that various PEG and ZnCl₂ concentrations have on the precipitation of the an antibody in a composition at pH 8.0.
FIG. 11 depicts the effect that various PEG and ZnCl₂ concentrations have on the precipitation of the an antibody in a composition at pH 9.0.
FIG. 12 depicts size exclusion analysis of Ab F following precipitation with PEG 3350 and ZnCl₂, in the presence of imidazole. The resolubilized fraction of Ab F was subsequently purified through an anion exchange step and an hydrophobic interaction chromatography step.
FIG. 13 depicts dead-end (Nutsche) filtration of Ab F precipitate with varying concentrations of body feed. Differential pressure (psi) vs. throughput (L/m²) shown in (A), and Volume (ml) of filtrate vs. time (min) shown in (B).
FIG. 14 depicts size-exclusion analysis of washed Ab F precipitate cakes at increasing diavolumes (DV) of wash solution. A low-molecular weight impurity (LMW) is highlighted by the arrows.
FIG. 15 depicts low molecular weight removal by precipitation with PEG and ZnCl₂, in presence and absence of imidazole. The 10-fold concentrated Ab F HCCF is shown in (A). Precipitation of Ab F with PEG 3350 and ZnCl₂ in the absence of imidazole is shown in (B) (LMW levels ∼6%), and precipitation of Ab F with 3350 and ZnCl₂ in the presence of imidazole is shown in (C) (LMW levels <1%).

### DETAILED DESCRIPTION OF THE INVENTION

The addition of a combination of both a polyalkylene glycol and a transition metal was found to improve precipitation of biomacromolecules in a composition. This precipitation of the biomacromolecule resulted in a separation of the biomacromolecule from impurities present in the composition. The present invention is related to method of isolating a biomacromolecule in a composition containing an impurity, the method comprising (a) adding a polyalkylene glycol to the composition; (b) adding a transition metal to the composition; and (c) separating the biomacromolecule from the impurity.

It is to be noted, unless otherwise clear from the context, that the term "a" or "an" entity refers to one or more of that entity; for example, "a protein," is understood to represent one or more proteins. As such, the terms "a" (or "an"), "one or more," and "at least one" can be used interchangeably herein.

The terms "isolating" and "isolation" refer to separating a biomacromolecule from at least one other undesired component or impurity found in the composition. The term "isolating" includes "purifying" and "clarifying." No particular level of isolation of a biomacromolecule is required, however in some embodiments, at least 50%, 70%, 80%, 90%, 95%, 96%, 97%, 98% or 99% (w/w) of an impurity is separated from the biomacromolecule. For example, in some embodiments, isolation would comprise separating the biomacromolecule from 80% of the host cell proteins (HCP) present originally in the composition.

The terms "clarifying" and "clarification" refer to the removal of large particles from a composition. For example, as applied to cellular cultures and growth media, the term "clarifying" refers to, e.g., the removal of prokaryotic and eukaryotic (e.g., mammalian) cells, lipids, and/or nucleic acids (e.g., chromosomal and plasmid DNA) from the cell culture.

The terms "purifying" and "purification" refer to separating the biomacromolecule from an impurity or other contaminants in the composition, regardless of the size of the impurity. Thus, the term purification would encompass "clarification," but it would additionally encompass impurities smaller in size than those removed during clarification, e.g., proteins, lipids, nucleic acids, and other forms of cellular debris, viral debris, contaminating bacterial debris, media components, and the like. No particular level of purification is required, however in some embodiments, at least 50%, 70%, 80%, 90%, or 95% (w/w) of an impurity is purified from the biomacromolecule. For example, in some embodiments, purification of a biomacromolecule would comprise separating the biomacromolecule from 80% of the HCP present originally in the composition.

The terms "synergy," "synergistic," or "synergistic effect" as used herein describe an effect which has a magnitude that is greater than additive. In some embodiments of the present invention, the use of both a polyalkylene glycol and a transition metal in concert provides synergistic product recovery or synergistic precipitation of a biomacromolecule. For example, if use of 3% (w/v) polyalkylene glycol *alone* precipitated 5% of a biomacromolecule and use of a 10mM final concentration of a transition metal *alone* precipitated 5% of the same biomacromolecule, then the additive effect for precipitating the biomacromolecule with both 3% polyalkylene glycol and 10mM transition metal in concert would be 10% precipitation of the biomacromolecule. Hence, by comparison, a synergistic effect when using both 3% polyalkylene glycol and 10mM transition metal in concert would be precipitation of the biomacromolecule to any extent greater than 10%.

Various polyalkylene glycols can be used. In some embodiments, the term polyalkylene can be a compound of the general formula I:

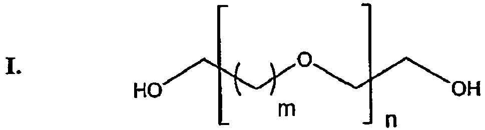

In some embodiments, n can be about 10 to about 5,000, about 20 to about 2,500, about 50 to about 2,000, or about 100 to about 500, and m is about 1 to about 10. Additional polyalkylene glycol molecules have been described previously. Suitable examples include polypropylene glycols, such as those described in U.S. Pat. No. 5,643,575. Other polyalkylene glycols useful in the methods of the invention are described in Shearwater Polymers, Inc. catalog "Polyethylene Glycol and Derivatives 2001".

In some embodiments, heteropolymers can be used, wherein different alkyl substituents are contained in the polyalkylene molecule, e.g., a polyalkylene molecule of Formula II: 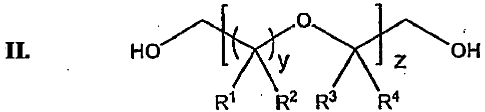 where R¹, R², R³, R⁴ can independently be a hydrogen or substituted or unsubstituted alkyl, arylalkyl, cycloalkylalkyl, heterocycloalkyl, heteroarylalkyl, phosphate, phosphoalkyl, sulfate or sulfoalkyl, and wherein each z subunit can be the same or different.

In some embodiments, y can be about 1 to about 10. In some embodiments, the value of y, can optionally vary for each z subunit. In some embodiments, z can be about 10 to about 5,000, about 20 to about 2,500, about 50 to about 2,000, or about 100 to about 500.

In some embodiments, the polyalkylene glycol is water-soluble at room temperature. In some embodiments, the polyalkylene glycol is selected from the group consisting of polypentyleneglycol, polybutyleneglycol, polypropylene glycol, or polyethylene glycol, wherein any of the alkyl substituents are optionally unsubstituted or substituted with an alkyl, arylalkyl, cycloalkylalkyl, heterocycloalkyl, heteroarylalkyl, phosphate, phosphoalkyl, sulfate or sulfoalkyl. In some embodiments, the polyalkylene glycol is a polyethylene glycol.

Various molecular weights of polyalkylene glycols can be used. Although polyalkylene glycols can vary substantially in average molecular weight, in some embodiments, the polyalkylene glycol has an average molecular weight of from about 1,000 Da to about 100,000 Da. In some embodiments, the polyalkylene glycol has a molecular weight of between 1,000 Da and 50,000 Da, about 2,000 Da to about 25,000 Da, about 3,000 Da to about 20,000 Da, or about 4,000 to about 10,000. In some embodiments, the polyalkylene glycol has a molecular weight of about 3,000 Da, 4,000 Da, 5,000 Da, 6,000 Da, 7,000 Da, 8,000 Da, or 9,000 Da. In some embodiments, the polyalkylene glycol has a molecular weight of between 2,000 Da and 15,000 Da.

In some embodiments wherein the polyalkylene glycol is polyethylene glycol, the polyethylene glycol has a molecular weight between 1,000 Da and 50,000 Da, about 2,000 Da to about 25,000 Da, about 3,000 Da to about 20,000 Da, or about 4,000 to about 10,000. In some embodiments, the polyethylene glycol has a molecular weight of about 3,000 Da, 4,000 Da, 5,000 Da, 6,000 Da, 7,000 Da, 8,000 Da, or 9,000 Da. In some embodiments, the polyethylene glycol has a molecular weight of between 2,000 Da and 15,000 Da.

Various concentrations of polyalkylene glycols can be used in the composition. The polyethylene can be added to the composition from a concentrated, or optionally pure, stock. The concentrated stock is diluted upon addition to the composition. In some embodiments, adding the polyalkylene glycol to the composition results in a composition having a polyalkylene glycol concentration of about 1% to about 40%, about 2% to about 35% (w/v), about 2.5% to about 30% (w/v), or about 3% to about 30% (w/v). In some embodiments, the polyalkylene glycol concentration is about 1%, 2%, 3%, 4%, 5%, 6%, 7%, 8%, 9%, 11%, 12%, 13%, 14%, or 15% (w/v) of the composition. In some embodiments, adding the polyalkylene glycol to the composition results in a composition having a polyalkylene glycol concentration of about 2.0% to about 10% (w/v). In some embodiments, adding the polyalkylene glycol to the composition results in a composition having a polyalkylene glycol concentration of about 0.5% to about 30% (w/v). In some embodiments, adding the polyalkylene glycol to the composition results in a composition having a polyalkylene glycol concentration of about 0.5% to about 2.5% (w/v). In some embodiments, the concentration of polyalkylene glycol used in combination with a transition metal is about 1%, 5%, 10%, 20%, 30%, 40%, 50%, 60%, or 70% less than the concentration of polyalkylene glycol used without the addition of a transition metal, to achieve an equivalent amount of separated biomacromolecule. In such embodiments, there can be reduced costs associated with the use of less polyalkylene glycols, and less residual waste is created after purification.

One of skill in the art will understand that different concentrations, molecular weights, or types of polyalkylene glycols may be more effective for different biomacromolecules. While not being bound by any methodology, a suitable concentration of polyalkylene glycol can be determined by adding various concentrations, molecular weights, or types of polyalkylene glycol to a composition comprising a transition metal and a biomacromolecule, and then determining the most efficient method of purifying the biomacromolecule.

Transition metals can be used in the present invention to increase separation of the biomacromolecule from the impurity. The term "transition metal" refers to any element in the d-block of the periodic table, including zinc, cadmium and mercury. Examples of transition metals include scandium, titanium, vanadium, chromium, iron, zinc, nickel, copper, cobalt and manganese. In some embodiments, the transition metal is zinc, nickel, copper, cobalt or manganese. In some embodiments, the transition metal is zinc. In some embodiments, more than one type of transition metal can be used. For example, in some embodiments, a combination of two or more of zinc, nickel, copper, cobalt and manganese can be used. In some embodiments, the transition metal is micronized to decrease the average size of the transition metal particles.

Various concentrations of transition metals in the composition are suitable for use in the present invention. One of skill in the art will recognize that various endogenous amounts of transition metals can be normally present in small amounts in the composition, e.g., the harvest feed (endogenous transition metals), and that various amounts of transition metals can be added to the harvest feed in accordance with the present invention (exogenous transition metals). In some embodiments, the concentration of the transition metals comprises both exogenous and endogenous cations. However, for practical purposes, since the amount of endogenous transition metals is usually relatively small compared to the amount of exogenous transition metals, the concentration of the transition metals can be calculated by simply considering the exogenous transition metals. In some embodiments, an aliquot of a transition metal from a concentrated (or pure) stock is added to the composition of the present invention to obtain the desired final concentration of the transition metal in the composition. In some embodiments, additional composition is added to a transition metal, in order to dilute the concentration of the transition metal in the composition. In some embodiments, the adding the transition metal to the composition results in a composition having a transition metal concentration of about 0.1 mM to about 1 M, about 0.1 mM to about 100 mM, about 0.2 mM to about 75 mM, about 0.5 mM to about 50 mM, or about 1 mM to about 20 mM. In some embodiments, the adding the transition metal to the composition results in a composition having a transition metal concentration of about 1.5 mM to about 15 mM, 2.5 mM to about 15 mM, or about 0.3 mM to about 7.0 mM. In some embodiments, the concentration of transition metal used in combination with a polyalkylene glycol is about 10%, 20%, 30%, 40%, 50%, 60%, or 70% less than the concentration of transition metal used without the addition of a polyalkylene glycol, to achieve an equivalent amount of separated biomacromolecule. In such embodiments, there can be reduced costs associated with the use of less transition metal, and less residual waste is created after purification.

One of skill in the art will recognize that different concentrations of transition metals may be more effective for different biomacromolecules. While not being bound by any methodology, a suitable concentration of transition metal can be determined by adding various concentrations of transition metals to a composition comprising a polyalkylene glycol and a biomacromolecule, and then determining the lowest concentration at which a maximum amount of biomacromolecule can be recovered.

One of skill in the art will recognize that the transition metal can exist in a salt form, e.g., a copper salt such as CuCl₂ can produce a copper cation when placed in an aqueous solution. Thus, as used herein, the phrase "adding a transition metal" would encompass not only the addition of the transition metal in its charged state, but also the addition of a salt or other compound that would produce a transition metal upon introduction into the composition.

In some embodiments, the transition metal is a salt (e.g., CuCl₂, NiCl₂,, ZnCl₂, MnCl₂, CuBr₂, NiBr₂, ZnBr₂, MnBr₂, CuSO₃, NiSO₃, ZnSO₃, and MnSO₃), or combinations of one or more of these cations or salts. In some embodiments, the transition metal is a halogenated salt, e.g., CuCl₃, NiCl₃, etc. In some embodiments, the transition metal is copper glycinate, nickel glycinate, zinc glycinate, or manganese glycinate. It is to be expected that certain transition metals may be more suitable for different biomacromolecules to be purified/isolated. However, one of skill in the art can easily and quickly test many transition metals to determine which transition metal achieves the maximum recovery of the biomacromolecule of interest.

In some embodiments, competitive ligands which affect metal ion binding in aggregation are useful in the present invention. Examples of such ligands include, without limitation, imidazole, glycine, tryptophan, cysteine, histidine, histamine, ammonium chloride. (*See* Kagedal, L, "Immobilized Metal Ion Affinity Chromatography" in Protein Purification: Principles, High Resolution Methods, and Applications (Janson, J-C,1998) and Przbycien et al. "A Model for Metal Affinity Precipitation" J. Coli. Int. Sci (1996)).

The term "composition" in the present invention refers to a mixture of at least one molecule of the biomacromolecule and optionally at least one impurity, wherein the impurity and the biomacromolecule are not the same. In some embodiments, the composition comprises a biomacromolecule, a cellular host organism (e.g., mammalian cells), and a growth media sufficient for propagating the host organism and allowing expression of the biomacromolecule of interest. The selection and use of growth medium are known to those in the art. In some embodiments, the growth media is a cell culture media. Cell culture media vary according to the type of cell culture being propagated. In some embodiments, the cell culture media is a commercially available media. In some embodiments, the composition comprises a growth media which contains e.g., inorganic salts, carbohydrates (e.g., sugars such as glucose, galactose, maltose or fructose) amino acids, vitamins (e.g., B group vitamins (e.g., B12), vitamin A vitamin E, riboflavin, thiamine and biotin), fatty acids and lipids (e.g., cholesterol and steroids), proteins and peptides (e.g., albumin, transferrin, fibronectin and fetuin), serum (e.g., compositions comprising albumins, growth factors and growth inhibitors, such as, fetal bovine serum, newborn calf serum and horse serum), trace elements (e.g., zinc, copper, selenium and tricarboxylic acid intermediates) and combinations thereof Examples of growth medias include, but are not limited to, basal media (e.g., MEM, DMEM, GMEM), complex media (RPMI 1640, Iscoves DMEM, Leibovitz L-15, Leibovitz L-15, TC 100), serum free media (e.g., CHO, Ham F10 and derivatives, Ham F12, DMEM/F12). Common buffers found in growth media include PBS, Hanks BSS, Earles salts, DPBS, HBSS, and EBSS. Media for culturing mammalian cells are well known in the art and are available from, e.g., Sigma-Aldrich Corporation (St. Louis, MO), HyClone (Logan, UT), Invitrogen Corporation (Carlsbad, CA), Cambrex Corporation (E. Rutherford, NJ), JRH Biosciences (Lenexa, KS), Irvine Scientific (Santa Ana, CA), and others. Other components found in growth media can include ascorbate, citrate, cysteine/cystine, glutamine, folic acid, glutathione, linoleic acid, linolenic acid, lipoic acid, oleic acid, palmitic acid, pyridoxal/pyridoxine, riboflavin, selenium, thiamine, and transferrin. One of skill in the art will recognize that there are modifications to growth media which would fall within the scope of this invention.

In some embodiments, the composition further comprises a harvest feed. The term "harvest feed" refers to a media in which cells are present in immediately before harvesting, or a media in which harvested cells are placed immediately after harvesting and into which the cells are resuspended. A harvest feed can include any of the compositions listed above for growth media, or other media suitable for resuspending the harvested cells or cellular fractions. For example, in some embodiments, the harvest media may contain water, a buffer, osmotic agents, anti-degradation agents, etc. In some embodiments, the invention is related to a method of separating a biomacromolecule from a harvest feed.

The biomacromolecule can be isolated from a composition comprising cell culture, wherein the cell culture comprises growth media and various eukaryotic cells, e.g., mammalian cells. The mammalian cells of the present disclosure, include any mammalian cells that are capable of growing in culture. Exemplary mammalian cells include, e.g., CHO cells (including CHO-K1, CHO DUKX-B11, CHO DG44), VERO, BHK, HeLa, CV1 (including Cos; Cos-7), MDCK, 293, 3T3, C127, myeloma cell lines (especially murine), PC12, HEK-293 cells (including HEK-293T and HEK-293E), PER C6, Sp2/0, NS0 and W138 cells. Mammalian cells derived from any of the foregoing cells may also be used.

The biomacromolecule can be isolated from a cell culture comprising growth media and various prokaryotic or non-mammalian cells, e.g., E. coli, Bacillus subtilis, Salmonella typhimurium and various species within the genera Pseudomonas, *e.g., P. aeruginosa,* yeast cells, *e.g., Saccharomyces, Pichia, Hansenula, Kluyveromyces, Schizosaccharonryces, Schwanniomyces and Yarrowia,* insect cells, *e.g., Trichoplusia, Lipidotera, Spodoptera, Drosophila* and Sf9, or plant cells, *e.g., Arabidopsis.* One of skill in the art can select an appropriate prokaryotic or non-mammalian cell depending on the biomacromolecule of interest.

The terms "biological biomacromolecule" or "biomacromolecule" as used herein refer to an antibody, e.g., a monoclonal antibody or a polyclonal antibody.

As used herein, the term "protein" is intended to encompass a singular "protein" as well as plural "proteins." Thus, as used herein, terms including, but not limited to "peptide," "polypeptide," "amino acid chain," or any other term used to refer to a chain or chains of amino acids, are included in the definition of a "protein," and the term "protein" may be used instead of, or interchangeably with, any of these terms. The term further includes proteins which have undergone post-translational modifications, for example, glycosylation, acetylation, phosphorylation, amidation, derivatization by known protecting/blocking groups, proteolytic cleavage, or modification by non-naturally occurring amino acids. Proteins also include polypeptides which form multimers, e.g., dimers, trimers, etc. The term protein also includes fusions proteins, e.g., a protein that is produced via a gene fusion process in which a protein (or fragment of a protein) is attached to an antibody (or fragment of antibody). Examples of fusion proteins of the present disclosure include disulfide-linked bifunctional proteins comprised of linked Fc regions from human IgG1 and human IgE; and lymphotoxin beta receptor immunoglobulin G1.

Antibodies can be purified according to the method of the present invention. The term "antibody" refers to polyclonal, monoclonal, multispecific, human, humanized or chimeric antibodies, single chain antibodies, Fab fragments, F(ab')2 fragments, fragments produced by a Fab expression library, anti-idiotypic (anti-Id) antibodies), and epitope-binding fragments of any of the above. In some embodiments, the term "antibody" refers to a monoclonal antibody. The term "antibody" also refers to immunoglobulin molecules and immunologically active portions of immunoglobulin molecules, i.e., molecules that contain an antigen binding site that immunospecifically binds an antigen. The immunoglobulin molecules that can be purified by the method of the invention can be of any type (e.g., IgG, IgE, IgM, IgD, IgA and IgY), class (e.g., IgG1, IgG2, IgG3, and IgG4) or subclass of immunoglobulin molecule. Antibodies of the present disclosure also include chimeric, single chain, and humanized antibodies. Examples of antibodies of the present disclosure include commercialized antibodies, such as natalizumab (humanized anti-a4 integrin monoclonal antibody), humanized Anti-Alpha V Beta 6 monoclonal antibody, humanized anti-VLA1 IgG1 kappa monoclonal antibody; huB3F6 (humanized IgG1/kappa monoclonal antibody).

Antibodies purified by the method of the invention may be from any animal origin including birds and mammals. In some embodiments; the antibodies purified by the method of the invention are human, murine (e.g., mouse and rat), donkey, ship rabbit, goat, guinea pig, camel, horse, or chicken antibodies. As used herein, "human" antibodies include antibodies having the amino acid sequence of a human immunoglobulin and include antibodies isolated from human immunoglobulin libraries or from animals transgenic for one or more human immunoglobulin and that do not express endogenous immunoglobulins. See, e.g., U.S. Pat. No. 5,939,598 by Kucherlapati et al. In some embodiments, the term "antibody" includes, but is not limited to, IgG1, IgG2, IgG3, and IgG4 antibodies, including commercialized antobodies, such as natalizumab (TYSBARI®, Elan Pharmaceuticals, San Diego, CA).

Antibodies that can be purified by the method of the invention include, e.g., native antibodies, intact monoclonal antibodies, polyclonal antibodies, multispecific antibodies (e.g., bispecific antibodies) formed from at least two intact antibodies, antibody fragments (e.g., antibody fragments that bind to and/or recognize one or more antigens), humanized antibodies, human antibodies (Jakobovits et al., Proc. Natl. Acad. Sci. USA 90:2551 (1993); Jakobovits et al., Nature 362:255-258 (1993); Bruggermann et al., Year in Immunol. 7:33 (1993); U.S. Patent Nos. 5,591,669 and 5,545,807), antibodies and antibody fragments isolated from antibody phage libraries (McCafferty et al., Nature 348:552-554 (1990); Clackson et al., Nature 352:624-628 (1991); Marks et al., J. Mol. Biol. 222:581-597 (1991); Marks et al., Bio/Technology 10:779-783 (1992); Waterhouse et al., Nucl. Acids Res. 21:2265-2266 (1993)). The antibodies purified by the method of the invention may be recombinantly fused to a heterologous polypeptide at the Nor C-terminus or chemically conjugated (including covalently and non-covalently conjugations) to polypeptides or other compositions. For example, antibodies purified by the method of the present invention may be recombinantly fused or conjugated to molecules useful as labels in detection assays and effector molecules such as heterologous polypeptides, drugs, or toxins. See, e.g., PCT publications WO 92/08495; WO 91/14438; WO 89/12624; U.S. Pat. No. 5,314,995; and EP 396,387.

In some embodiments, the biomacromolecule or composition of the present disclosure is pharmaceutically acceptable. "Pharmaceutically acceptable" refers to a biomacromolecule or composition that is, within the scope of sound medical judgment, suitable for contact with the tissues of human beings and animals without excessive toxicity or other complications commensurate with a reasonable benefit/risk ratio.

The term "soluble" refers to the propensity of a protein to substantially localize to the hydrophilic or aqueous-based environments of a cellular host, e.g., the cytoplasm, periplasm or extracellular medium. Thus, during cellular fractionation, a soluble protein would generally be substantially isolated with the cytoplasmic, periplasmic, or extracellular components of a host cell. In some instances, a soluble protein is water soluble in the absence of detergents. One of skill in the art will recognize that neither the cellular localization of a polypeptide, nor the cellular fractionation of a protein, is absolute. Thus, the phrase "substantially localize" refers to a protein in which 50%, 70%, 75%, 80%, 85%, 90%, 95%, or 99% of the protein is in the designated cellular location, e.g., cytoplasm, periplasm, or extracellular medium. In some instances, the protein can be a membrane protein, or a membrane associated protein.

The present invention is useful to isolate a biomacromolecule from a composition containing an impurity. In some embodiments, the composition comprises eukaryotic cellular material. In some embodiments, the impurity is selected from the group consisting of a growth media component, protein, lipid, nucleic acid, ribonucleic acid, and combinations thereof.

The term "impurity" refers to one or more components of the composition that is different from the biomacromolecule of the present disclosure In some embodiments, the impurity can include an intact mammalian cell (e.g., Chinese hamster ovary cells (CHO cells) or murine myeloma cells (NSO cells)), or partial cells, e.g., cellular debris. In some embodiments, the impurity comprises a protein (e.g., soluble or insoluble proteins, or fragments of proteins, such as HCP), lipid (e.g, cell wall material), nucleic acid (e.g., chromosomal or extrachromosomal DNA), ribonucleic acid (t-RNA or mRNA), or combinations thereof, or any other cellular debris that is different from the biomacromolecule of interest. In some embodiments, the impurity can originate from the host organism that produced or contained the biomacromolecule of interest. For example, an impurity could be a cellular component of a prokaryotic or eukaryotic cell (e.g., cell wall, cellular proteins, DNA or RNA, etc.) that expressed a protein of interest. In some embodiments, the impurity is not from the host organism, e.g., an impurity could be from the cell culture media or growth media, a buffer, or a media additive. The impurity as used herein can include a single undesired component, or a combination of several undesired components.

Various means can be used to separate the biomacromolecule from one or more impurities. Examples of means of separating the biomacromolecule from an impurity include, without limitation, precipitation, immunoprecipitation, chromatography, filtration, ultrafiltration, diafiltration, Nutshe filtration, crossflow filtration, centrifugation, and combinations thereof.

In some embodiments, the separating of the biomacromolecule from the impurity is achieved by precipitation, i.e., the words "separating" and "precipitating" can be interchangeable. Precipitation refers to the formation of a solid composition in a solution. In some embodiments, the solid is of a greater density than the solution and "falls" out of the solute phase, sinking to the bottom of the solution (sedimentation). In some embodiments, the precipitation can be enhanced or accelerated by centrifugation.

In some embodiments, upon addition of the transition metal and the polyalkylene glycol to the composition, the biomacromolecule substantially precipitates out of the composition. In some embodiments, the impurity is simply decanted from the precipitated biomacromolecule, thereby isolating the biomacromolecule. In some embodiments, 50%, 60%, 70%, 80%, 85%, 90% 92%, 94%, 95%, 96%, 97%, 98% or 99% of the biomacromolecule is precipitated from the composition.

In some embodiments, the separating of the biomacromolecule from the impurity is achieved by the use of a filter. The term "filtration" or "filtering" refers to the process of removing suspended particles from a composition by passing the composition through one or more semi-permeable membranes (or medium) of a specified pore size diameter. The term "permeate stream" when referring to filtration, refers to the fraction of the composition that passes through the filter pores during filtration. The term "solid stream" when referring to filtration, refers to the fraction of the composition that remains on the filter or that does not pass through the filter pores during filtration. In some embodiments, after filtration the biomacromolecule is substantially in the permeate stream (i.e., it passes through the filter pores and is collected), while an impurity (e.g., cellular debris, DNA, and/or HCP) is substantially in the solid stream. In some embodiments, after filtration the biomacromolecule is substantially in the solid stream, while an impurity is substantially in the permeate stream. In some embodiments, "bench scale" filtration can be used to predict appropriate conditions for industrial scale filtration.

Suitable filter types, chemistries, and module configurations for purifying particular biomacromolecules are known to those in the art and can be selected based on various factors, e.g., the amount and size of the components of the composition to be filtered, the volume of the composition to be filtered, and the cell density and viability of the composition to be filtered. See, e.g., Reynolds T, Boychyn M, Sanderson T, Bulmer M, More J, Hoare, M, Biotechnology and Bioengineering, 83(4), pp. 454-464 (2003). In some embodiments, filters, such as membrane filters, plate filters, cartridge filters, bag filters, pressure leaf filters, rotary drum filters or vacuum filters can be used. In some embodiments, a depth filter or a cross filter is used. The types of crossflow filter modules that apply in the present invention include hollow fiber, tubular, flat plate (plate-and-frame), spiral wound, or vortex flow (e.g., rotating) filter geometries. In some embodiments, a tangential flow filter is used. In some embodiments, hollow fibers, tubular, and flat-sheet membrane modules were utilized in a tangential-flow (cross-flow) mode. Commercially available filters that can be employed are manufactured for various manufacturing vendors such as Millipore Corporation (Billerica, MA), Pall Corporation (East Hills, NY), GE Healthcare Sciences (Piscataway, NJ), and Sartorius Corporation (Goettingen, Germany). In some embodiments, a dead-end filter is used. Commercially available dead-end filters that can be employed are manufactured for various manufacturing vendors such as Millipore Corporation (Billerica, MA), Pall Corporation (East Hills, NY), GE Healthcare Sciences (Piscataway, NJ), and Sartorius Corporation (Goettingen, Germany).

The pore diameter in the filters of the present disclosure can vary according to the type of biomacromolecule being isolated and the type of impurities present in the composition. In some embodiments, the filter pore diameters can be 0.1 µm to 4.0 µm, 0.2 µm to 2.0 µm, or 0.2 µm to 1.5 µm in diameter.

Movement of a composition, such as a harvest feed, through a filter during filtration generates a transmembrane pressure resulting from membrane resistance. As the membrane surface becomes accumulated (or polarized) with cellular material, there is an increased resistance to flow across the membrane at a constant flowrate, thus causing the driving force or transmembrane pressure to increase. If the amount of cellular material near the surface of the membrane is reduced, or if the membrane is less polarized, the transmembrane pressure tends to remain substantially constant Methods to calculate transmembrane potential are know to those in the art, and include the use of pressure transducers or gauges. In some embodiments of the present invention, the transmembrane pressure can be calculated by taking the difference between the average of the feed and solid stream outlet pressure and the permeate stream pressure.

Generally, during filtration of a composition, e.g., a harvest feed, the transmembrane pressure of a filter increases significantly as more of the composition is loaded onto the filter. For example, in some embodiments the transmembrane pressure increases 5 psi, 7 psi, 10 psi, 15 psi or 20 psi or greater from the start of the filtration process (when the first amount of the composition is placed in the filter) to the end of the filtration process (typically following a 7-10x concentration of cellular material and a 3-5x diafiltration) as the pores of the filter become clogged. Thus, the term "substantially constant" where referring to the transmembrane pressure, refers to transmembrane pressures that do not increase greater than 4 psi, 3 psi, or 2 psi over the course of filtration. In some embodiments, the present invention is directed to a method of purifying a biomacromolecule in a composition, the method comprising (a) adding a polyalkylene glycol to the composition; (b) adding a transition metal to the composition; and then (c) filtering the composition through a membrane, the filtering resulting in a transmembrane pressure, wherein the transmembrane pressure remains substantially constant during the filtering.

In some embodiments, the method of the present invention decreases protein filter rejection. The term "protein filter rejection" can be exemplified by the equation R = (1-[C_{P}/C_{R}]), wherein R represents protein filter rejection coefficient, C_{P} is the instantaneous permeate concentration of the biomacromolecule of interest, and C_{R} is the instantaneous solid concentration of the biomacromolecule of interest. In some embodiments, the present invention is directed to a method of isolating a biomacromolecule in a composition comprising adding a polyethylene glycol to the composition, adding a transition metal to the composition, and filtering the biomacromolecule, wherein the value of the protein filter rejection coefficient is lower for a given volumetric throughput relative to a biomacromolecule in a composition without polyalkylene glycol and/or transition metal addition.

When isolating biomacromolecules, in some embodiments large volumes of a composition (e.g., harvest feed) can be present, e.g., during the commercial manufacturing processes. Large volumes present several challenges for purification processes. For example, the effect that a small change in flow rate through a filter has on the recovery of an isolated biomacromolecule is amplified when large volumes are used. Likewise, when using large volumes, the effect that an increase in cell density in a harvest feed has on product recovery is also amplified. Thus, the use of large volumes of a composition present unique problems that are amplified and have greater ramifications relative to the use of smaller volumes. Thus, in some embodiments the present invention is directed to a method of isolating a biomacromolecule present in a large volume of a composition. The term "large volume" refers to volumes associated with the commercial and/or industrial production of a biomacromolecule. In some embodiments, the term "large volume" refers to 10 to 30,000 liters, 20 to 20,000 liters or 50 to 15,000 liters.

In some embodiments, the method of the present invention comprises separating the biomacromolecule from an impurity by subjecting the composition to a centrifugal force (i.e., centrifugation), where the centrifugation forms a supernatant and a pellet. Various forms of centrifugation are known in the art. See, e.g., Boychyn M, Doyle W, Bulmer M, More J, Hoare M, Biotechnology and Bioengineering, 69(1), pp. 1-10 (2000). In some embodiments, the centrifugation forms a supernatant substantially free of an impurity (cells or cellular debris) and a concentrated cell/cellular debris pellet. The term "pellet" or "solid cake," when referring to centrifugation, refers to the fraction of the composition that is precipitated (or pelleted) during centrifugation to form a cell/cell debris mass. The term "supernatant," refers to the fraction of the composition that is not precipitated (or pelleted) during centrifugation, for example, the fraction of the composition that remains in an aqueous phase in the composition. In some embodiments, after centrifugation the biomacromolecule is substantially in the supernatant (i.e., it remains substantially suspended in the liquid fraction of the composition). In some embodiments, after centrifugation the biomacromolecule invention is substantially in the pellet (e.g., it is precipitated out of solution or is present in a pellet resulting from the centrifugation). In some embodiments, 50%, 60%, 70%, 80%, 85%, 90% 92%, 94%, 95%, 96%, 97%, 98% or 99% (w/w) of the biomacromolecule is in the pellet. In some embodiments, a density gradient is used to separate the biomacromolecule from the impurity. Thus, in some embodiments both the biomacromolecule and the impurity remain in the supernatant after centrifugation, albeit at different densities and thus different locations in the centrifugation apparatus.

Various centrifugation apparatuses can be used. In some embodiments, the centrifugation can be accomplished by disc stack centrifugation. In some embodiments, "bench scale" filtration can be used to predict appropriate conditions for industrial scale filtration. Centrifugation variables can be varied to achieve optimal isolation of the biomacromolecule of interest. For example, in some embodiments, various rotational speeds or flow rates can be used to increase the quality of biomacromolecule recovery, and/or the quantity of biomacromolecule recovery.

The present invention can be useful when isolating a biomacromolecule from a bioreactor containing cell culture with an initial high density of biological material. For example, in some high density cultures, the transmembrane pressure often drops significantly across the filter, presumably as a result of fouling of the membrane surface due to the high concentration of cellular material. Increased filter fouling in turn can adversely impact the quantity of the biomacromolecule recovered, lowering yields, and resulting in relatively higher impurity levels in the permeate stream. In some cases, the transmembrane pressure can increase to values beyond the mechanical capabilities of the filter, thus causing the operation to stop before completion and resulting in significantly lower product yield. In order to reduce filter transmembrane pressure, increase protein recovery in the solid stream, and decrease the amount of impurities in the solid stream, in some embodiments, the method of the present invention comprises addition of polyalkylene glycol and a transition metal before filtration, causing flocculation of large cells and cellular debris along with precipitation of other impurities (such as DNA). Flocculation of impurities (cells, cellular debris and DNA) into large particles can improve mass transfer of the composition near the surface of the filter, thus reducing transmembrane pressure across the filter at a predetermined permeate stream flux or flowrate.

In some embodiments, it is beneficial or desirable to harvest a biomacromolecule from a high cell density composition (e.g., harvest feed). High cell density compositions present unique problems relative to normal cell density compositions. For example, high cell density compositions can have higher amounts of impurities present in the composition, thereby increasing the amount of impurities that need to be removed during the purification process. For example, a higher cell density composition can foul a filter more quickly, thereby prohibiting filtration of the composition. In some embodiments, high cell density compositions require the use of more filters, or filters with larger surface areas. Both of these requirements can result in greater costs associated with filtration and/or loss of product. In the present invention, polyalkylene glycol and a transition metal are added to the composition, thereby removing some impurities, and allowing the purification of higher cell density compositions. Thus, some embodiments in the present irivention are directed to a method of isolating a biomacromolecule present in a high cell density composition. The term "high cell density" generally refers to cell densities in a harvest feed of about 1 x 10⁵ to 3.5 x 10⁷, about 1.0 x 10⁶ to about 1.0 x 10⁷, or about 5.0 x 10⁶ to about 9.0 x 10⁶ cells per ml for mammalian cells. Of course, one of skill in the art will appreciate that various cells traditionally grow at different cell densities. Thus, in some embodiments, "high cell density" cell cultures refers to cell cultures containing cells at a density higher than the density traditionally practiced for that cell line.

In some embodiments of the present invention, the pH of the composition is adjusted prior to separating the biomacromolecule from the impurity. For example, in some embodiments, the pH of the composition is adjusted to a pH lower than that of the harvest feed. Compositions of the present disclosure, e.g., those comprising a harvest feed, generally have a pH of about 6.0 to about 8.0, about 6.5 to about 7.5 or about 6.8 to about 7.2 without adjustment. In some embodiments, any pH lower than the pH of the harvest feed can be used in the isolation of the present invention. In some embodiments, the pH of the composition is lowered to a pH within a range of about 1.0 to about 6.0, about 2.0 to about 5.5, about 3.0 to about 6.5, about 3.0 to about 5.0, about 4.0 to about 5.0, about 4.0 to about 4.7, about 4.3 to about 5.0, or about 4.7 to about 5.0. In some embodiments, the pH of the composition is lowered to within a range of about 4.0 to about 4.7. In some embodiments, the pH can be lowered to a pH of about 3.5. For some biomacromolecules, a pH lower than 3.5 results in denaturation or instability of the biomacromolecule of interest, and thus is not desirable. In some embodiments, the pH of the composition is raised to a pH within a range of about 6.0 to about 10.0, about 6.5 to about 9.5, about 7.0 to about 9.0, about 7.5 to about 8.5, about 7.0 to about 8.0, or about 7.5. In some embodiments, the pH of the composition is raised to within a range of about 7.0 to about 9.0. In some embodiments, the pH can be raised to a pH of about 8.0 or about 9.0.

The pH of the composition of the present disclosure can be adjusted by various means. In some embodiments, the pH is lowered by addition of an acid or a base to the composition. Suitable acids include, but are not limited to, strong acids such as perchloric acid (HClO₄), hydroiodic acid (HI), hydrobromic acid (HBr), hydrochloric acid (HCl), nitric acid (HNO₃), sulfuric acid (diprotic) (H₂SO₄), or weak acids such as acetic acid (CH₃COOH) (e.g., glacial acetic acid), citric acid (C₆H₈O₇), formic acid (HCOOH), hydrocyanic acid (HCN), hydrogen sulfate ion (HSO₄⁻), or combinations of any of the acids listed above. Suitable bases include, but are not limited to, strong bases such as potassium hydroxide (KOH), barium hydroxide (Ba(OH)₂), caesium hydroxide (CsOH), sodium hydroxide (NaOH), strontium hydroxide (Sr(OH)₂), calcium hydroxide (Ca(OH)₂), lithium hydroxide (LiOH), rubidium hydroxide (RbOH), or weak bases such as ammonia, or combinations of any of the bases listed above.

In some embodiments, the pH of the composition can be adjusted by use of buffers, such as phosphate buffers (e.g., sodium and potassium phosphates), bicarbonate buffers, citrate buffers, borate buffers, acetate buffers, tromethamine buffers, HEPES buffers, and combinations thereof.

In some embodiments, the addition of a transition metal to the composition results in coprecipitation of the biomacromolecule of interest as well as the impurity, resulting in reduced purity of the biomacromolecule upon isolation. In some embodiments, addition of polyethylene glycol to the composition is suitable for increasing the recovery of the biomacromolecule of interest. The term "increased recovery" refers to a comparison of method of the present invention (with addition of both a polyalkylene glycol and a transition metal) relative to an identical method of purifying but without the addition of transition metal or polyalkylene glycol. For example, if Method A is the method of the present invention (except it does not comprise addition of transition metals to the harvest feed) and yields 100 mg of the biomacromolecule of interest, and Method B is identical to Method A (except Method B comprises addition of transition metals to the harvest feed) and yielded 110 mg of biomacromolecule, then it would be determined that Method B has an "increased selective recovery" of 10%. In some embodiments, the method of the present invention increases recovery of the biomacromolecule by greater than 3%, 4%, 5%, 6%, 7%, 8%, 9%, 10%, 15%, 20% or 25%. In some embodiments, the method of the present invention increases recovery up to 10%, 15%, 20%, 25%, 30% or 50%. In some embodiments, the addition of the polyalkylene glycol and transition metal increases the recovery of the biomacromolecule by greater than 3%. In some embodiments, the recovery of the polyalkylene glycol and transition metal increases the recovery of the biomacromolecule by greater than 10%.

In some embodiments of the invention, a polyalkylene glycol is added to the composition. The term some embodiments of the present invention, the polyalkylene glycol is a polyethylene glycol having a molecular weight of between 1,000 Da and 20,000 Da, the transition metal is zinc, the separating is performed by precipitating the biomacromolecule in the solution and then centrifuging the composition to isolate the precipitate, and the biomacromolecule is an antibody.

In some embodiments of the present invention, the polyalkylene glycol is a polyethylene glycol having a molecular weight of between 1,000 Da and 20,000 Da, the transition metal is zinc, the separating is performed by precipitating the biomacromolecule in the solution and then filtering the composition to isolate the precipitate, and the biomacromolecule is an antibody.

In some embodiments of the present invention, the method of the present invention is directed to a method of increasing robustness of a filtration process, the method comprising (a) adding a polyalkylene glycol to a composition; (b) adding a transition metal to the composition; and (c) filtering the composition through a membrane. The term "increase robustness" refers the ability to use a wider range of flow rates for a given filter while not increasing transmembrane pressure, impurity concentrations, or product loss. The term "increase robustness" also refers to the ability to filter a larger volume, or higher cell density, of harvest feed for a given filter while not increasing transmembrane pressure, impurity concentrations, or product loss.

In some embodiments of the present invention, the method is drawn to a method of clarifying a composition comprising a biomacromolecule, e.g., a harvest feed, prior to filtration, the method comprising (a) adding a polyalkylene glycol to the composition; (b) adding a transition metal to the composition; and (c) separating the biomacromolecule from an impurity in the composition. In some embodiments, clarifying a composition correlates with a decrease in turbidty, as measured by a turbidimeter, such as a Hach 2100AN Turbidimeter (Hach Co., Loveland, CO).

The steps of the method of the present invention can be ordered in various sequences. For example, in some embodiments of the present invention, the adding the polyalkylene glycol occurs before the adding a transition metal and the separating the biomacromolecule away from the impurity. In some embodiments, the polyalkylene glycol is added first, then the transition metal is added, and then the biomacromolecule is separated from an impurity. In other embodiments, the transition metal is added first, the polyalkylene glycol is added second, and then the biomacromolecule is separated. In some embodiments, one or more purification procedures may occur between either (a) the adding of the polyalkylene glycol, (b) the adding of transition metal, or (c) the separating the biomacromolecule away from the impurity. Alternatively, the steps (a), (b), or (c) of the method of the present invention can be contiguous, e.g., no additional purification procedures occur between steps (a), (b) and (c). However, when steps (a), (b) and (c) are contiguous, additional purification procedures can occur before or after steps (a), (b) and (c). In some embodiments, the polyalkylene glycol and the transition metal are mixed together to form a common stock, prior to adding to the composition. In some embodiments, the polyalkylene glycol and the transition metal are added simultaneously to the composition. In some embodiments, the polyalkylene glycol and the transition metal are added consecutively, in any order, to the composition.

In some embodiments, concentration of the biomacromolecule prior to precipitation is effective in reducing the quantity of polyalkylene glycol and/or transition metal required for precipitation and recovery of the biomacromolecule. For example, concentration prior to precipitation may reduce the quantity of polyalkylene glycol and/or transition metal by about 2 to about 40-fold. Ultrafiltration, microfiltration and other methods of biomacromolecule concentration are effective methods of concentration prior to precipitation.

In some embodiments, the precipitate may be washed to further remove impurities prior to resolubilization of the biomacromolecule. For example, the precipitate may be washed with one or more solutions (or combinations of solutions) including, without limitation; water or solutions containing a polyalkylene glycol (for example, at a concentration of about 0.5 % to about 5 % (w/v)), a transition metal (for example, at a concentration of about 0.5mM to about 5mM), a chelator (for example,EDTA), an aromatic heterocyclic compound (for example, imidazole), or a variety of buffered solutions. In some embodiments washing is accomplished by addition of a solution, settling of the precipitate in a solution, and/or decantation or siphoning a solution from the precipitate.

In some embodiments, the biomacromolecule retains one or more biological activities following precipitation and resolubilization. In some embodiments, the biomacromolecule retains one or more biological activities following precipitation, washing, and resolubilization. The term "biological activity" refers to the naturally occurring function of the biomacromolecule ("naturally occurring" can also include aberrant or altered functions introduced by way of mutations in a biomacromolecule such as a protein, antibody, or enzyme). An example of one biological activity for an antibody includes specific binding of the antibody to an antigen. An example of biological activity for an enzyme includes the specific enzymatic or catalytic activity of the enzyme. In some embodiments, the biomacromolecule retains biological activity in a range of about 50 % to about 100 % (compared to the specific activity of the biomacromolecule prior to precipitation or prior to precipitation and washing). For example, the biomacromolecule may retain about 50%, about 60%, about 70%, about 80%, about 90%, about 95%, or about 100% of its specific biological activity prior to precipitation or prior to precipitation and washing.

In some embodiments, the pH of the solution is lowered to further effect resolubilization and/or dissassociation of a transition metal (such as zinc) from the biomacromolecule.

### Examples

The invention will be described in greater detail by way of specific examples. The following examples are offered for illustrative purposes, and are not intended to limit the invention in any manner. Those of skill in the art will readily recognize a variety of non-critical parameters that can be changed or modified to yield alternative embodiments in accordance with the invention.

### EXAMPLE 1

To investigate the precipitation of various antibodies and other proteins from cell culture compounds, several methods were tested and include: (1) precipitation using polyethylene glycol (PEG) 3350 alone in a single addition. (2) precipitation using zinc chloride alone in a single addition, (3) precipitation using a combination of PEG and zinc chloride in a single addition, (4) precipitation using PEG alone in incremental cuts *(i.e.,* incremental additions of PEG), and (5) precipitation using a combination of PEG and zinc in incremental cuts *(i.e.,* incremental additions of PEG and/or zinc).

To investigate the precipitation of antibodies Ab C, Ab D, and Ab E, using PEG alone in a single cut, a clarified harvest feed containing the antibody was adjusted to pH 8.0 with 2M Tris base. The harvest feed was then adjusted to a temperature of between 2-8°C, and held at that temperature throughout the purification process. The harvest feed was then filtered through a 0.22 µm filter. A 70% PEG 3350 stock was added to the filtered material to reach a 10% PEG 3350 concentration. The material was stirred for 10 minutes, and held for 1 hour. The 10% PEG composition was then centrifuged at 3,000 x g for 10 minutes, after which the supernatant was decanted. The resulting pellet was resuspended in a PEG-Zn resuspension buffer by rocking/mixing for 1 hour. The resuspension buffer comprises a buffer and a chelating agent.

The harvest sample, resuspended sample, and supernatant were assayed by SEC-HPLC to determine purity and recovery, taking into account any dilution or concentration factors. Additionally, the harvest sample and resuspended sample were assayed for HCP and DNA content by Elisa and QPCR methods. The term "titer" refers to the concentration of the product in the feed stock, prior to purification. The results are presented in Table 1.

**TABLE 1**

| **Product** | **Titer** | **pH** | **% PEG 3350** | **Product recovery** | **Purity** | **DNA log reduction** | **HCP log reduction** |
|---|---|---|---|---|---|---|---|
| Ab D | 1.5 g/L | 8 | 10% | 90% | 99% | -- | -- |
| Ab E | 2.0 g/L | 8 | 10% | 92% | 92% | 1.0 | 1.9 |
| Ab C | 2.1 g/L | 8 | 10% | 87% | 94% | 1.0 | 1.9 |

### EXAMPLE 2

To investigate for the precipitation of antibodies Ab C, Ab D, Ab E using zinc chloride alone in a single cut, a clarified harvest feed containing the antibody was adjusted to pH 7.2 with 2M Tris base. The harvest feed was then adjusted to a temperature of between 2-8°C, and held at that temperature throughout the purification process. The harvest feed was filtered through a 0.22 µm filter. A 250 mM zinc chloride stock was added to the filtered material to reach a final concentration of 10 mM ZnCl₂. The material was stirred for 10 minutes, and held for 1 hour.

The 10 mM ZnCl₂ composition was then centrifuged at 3,000 x g for 10 minutes, after which the supernatant was decanted. The resulting pellet was resuspended in a PEG-Zn resuspension buffer, pH 7.2, by rocking/mixing for 1 hour.

The harvest sample, resuspended sample, and supernatant were assayed by SEC-HPLC to determine purity and recovery, taking into account any dilution or concentration factors. Additionally, the harvest sample and resuspended sample were assayed for HCP and DNA content by Elisa and QPCR methods. The results are presented in Table 2.

**TABLE 2**

| **Product** | **Titer** | **pH** | **% PEG 3350** | **ZnCl₂ (mM)** | **Product recovery** | **Purity** | **HCP log reduction** |
|---|---|---|---|---|---|---|---|
| AbD | 1.5 g/L | 8 | 5% | 10 | 93% | 96% | -- |
| AbE | 2.0 g/L | 8 | 5% | 20 | 90% | 86% | 0.5 |
| AbC | 2.1 g/L | 7 | 5% | 10 | 92% | 88% | 0.5 |

### EXAMPLE 3

To investigate the precipitation of antibodies Ab C and Ab F using a combination of PEG and zinc chloride in a single addition, the clarified harvest containing the antibody was adjusted to pH 7.2 with 2M Tris base. The harvest feed was then adjusted to a temperature of between 2-8°C, and held at that temperature throughout the purification process. The harvest feed was then filtered through a 0.22 µm filter.

The first cut consisted of adding 70% PEG 3350 stock to the filtered material to reach a concentration of 1.6% PEG, and adding 250 mM ZnCl₂ to the filtered material to reach a final concentration of 2.7 mM ZnCl₂. The material was then stirred for 10 minutes, and held for 1 hour. The resulting sample was then filtered ("cut 1 filtrate") through a 0.22 um filter, and the filtrate was kept.

The second cut was made by adding PEG to the filtrate sample to a final concentration of 5.5% and zinc chloride to a final concentration of 5 mM. This material was held for 1h.

The resulting precipitate was centrifuged at 3,000 x g for 10 minutes, after which the supernatant was decanted. The resulting pellet was resuspended in PEG-Zn resuspension buffer, pH 7.2, by rocking/mixing for 1 hour.

The harvest sample, first cut filtrate, supernatant and resuspended sample were assayed by SEC-HPLC to determine purity and recovery, taking into account any dilution or concentration factors. Additionally, the harvest sample and resuspended sample were assayed for HCP and DNA content by Elisa and QPCR methods. The results are presented in Table 3.

**TABLE 3**

| **Product** | **Titer** | **pH** | **% PEG 3350** | **Product recovery** | **Purity** |
|---|---|---|---|---|---|
| AbC | 10.0 g/L | 8 | 4% | 83% | 95% |
| AbF | 9.3 g/L | 8 | 8% | 90% | 85% |

### EXAMPLE 4

To investigate the precipitation of antibody Ab F using PEG alone in 2 incremental cuts, the clarified harvest containing the antibody was adjusted to pH 8.0 with 2M Tris base. The harvest feed was then adjusted to a temperature of between 2-8°C, and held at that temperature throughout the purification process. The harvest feed was then filtered through a 0.22 µm filter.

The first PEG cut consisted of adding 70% PEG 3350 stock to the filtered material to a reach a concentration of 4.75% PEG. The material was then stirred for 10 minutes, and then held for 1 hour. The resulting sample was then filtered ("cut 1 filtrate") through a 0.22 um filter, and the filtrate is kept.

The second cut was made by adding PEG to the filtrate sample to a final concentration of 10.5%. This material was held for 1hour and the resulting precipitate was centrifuged at 3,000 x g for 10 minutes. The resulting supernatant was decanted, and the resulting pellet was resuspended using the PEG-Zn resuspension buffer, at pH 4.0, by rocking/mixing for 1 hour.

The harvest sample, first cut filtrate, supernatant and resuspended sample were assayed by SEC-HPLC to determine purity and recovery, taking into account any dilution or concentration factors. Additionally, the harvest sample and resuspended sample were assayed for HCP and DNA content by Elisa and QPCR methods. The results are presented in Table 4.

**TABLE 4**

| **Product** | **Titer** | **pH** | **% PEG 3350** | **ZnCl₂ (mM)** | **Product recovery** | **Purity** |
|---|---|---|---|---|---|---|
| AbF | 9.3 g/L | 7.2 | 4% | 10 | 87% | 84% |

### EXAMPLE 5

The effect of titer (concentration) on reagent requirements for Ab F precipitation was examined. Harvested cell culture fluid (HCCF) of Ab F (initial concentration of 6.5 g Ab F/L of HCCF) was adjusted to pH 7.2 with 2M Tris Base at 2-8°C. The HCCF was concentrated using PELLICON-2® 50 kilo Dalton (kDa) cartridge (Millipore, Inc.) to either 32.5 g/L or 65 g/L; one sample at 65 g/L was then diafiltered (buffer exchanged) into 5mM Sodium Phosphate buffer at pH 7.2 at conductivity of approximately 7 mS/cm at 6°C. Four different samples were generated: one at 6.5 g/L Ab F in cell culture fluid (CCF), one at 32.5 g/L Ab F in CCF, one at 65 g/L Ab F in CCF, and one at 65 g/L in the sodium phosphate buffer. Zinc chloride and PEG 3350 were added to the required concentration to achieve desired yield for each respective sample. The experimental conditions are presented in Table 5 and the results of the experiment are presented in Table 6.

**Table 5**

| | **15 K Base Case** | | **15 K with 10 X UF** | |
|---|---|---|---|---|
| | **8% PEG** | **4% PEG+ 5mM Zn** | **4% PEG+ 5 Mm zN (no DF)** | **1.5% PEG + 2.5 mM Zn (7 mS/cm)** |
| **Operating Volume** | >15,000 | >15,000 | ∼1,500 L | ∼1,500 |
| **PEG required** | 1,383 | 691 KG | 69 KG | 17 KG |
| **Zinc required** | - | 24.5 KG | 1.24 KG | 0.59 KG |
| **EDTA required** | - | 44.7 KG | 44.7 KG | 13.5 KG |

**Table 6**

| **Product** | **Titer** | **pH** | **% PEG 3350** | **ZnCl₂ (mM)** | **Product recovery** | **Purity** |
|---|---|---|---|---|---|---|
| Ab F | 6.5 g/L | 7.2 | 4% | 10 | 93% | 96% |
| Ab F | 32.5 g/L | 7.2 | 4% | 5 | 92% | 82% |
| Ab F | 65 g/L | 7.2 | 4% | 5 | 90% | 83% |
| Ab F | 65 g/L | 7.2 (difiltered) | 1.5% | 2.5 | 92%% | 87% |

### EXAMPLE 6

The effects of various other precipitating agents on the precipitation of various IgG1 molecules were investigated. Cell cultures expressing either antibody Ab A or Ab B were incubated with various amounts of either polyethylene glycol, zinc chloride, ethanol, caprylic acid or rivanol. The amount of the antibody precipitated was then determined by either size exclusion chromatography (for zinc chloride, ethanol, caprylic acid and rivanol), or by Protein A HPLC. The results are presented in Table 7.

**TABLE 7**

| **Precipitating agent** | **Antibody** | **Best average product yield** | **Change in % Aggregate** | **% OD₂₈₀ removed** |
|---|---|---|---|---|
| PEG (6000) | Ab A | 68% | N.D. | 92.3% |
| | Ab B | 85% | N.D. | 92.0% |
| Ethanol | Ab A | 96.2% | 2.0% | 95.1% |
| | Ab B | 16.0% | 50.2% | 92.0% |
| Zinc | Ab A | 61.0% | 2.0% | 90.0% |
| | Ab B | 54.2% | 12.7% | 92.4% |
| Rivanol | Ab A | Did not ppt. | Did not ppt. | Did not ppt. |
| | Ab B | Did not ppt. | Did not ppt. | Did not ppt. |
| Caprylic Acid | Ab A | 91.5% | None detected | 2.0% |
| | Ab B | 18.5% | None detected | 5.8% |

The above data suggests that the presence of PEG, ethanol, and zinc increases the monomer yield, as well as removal of cellular material (as measured by OD₂₈₀). The data suggests that caprylic acid removes the monomer yield, but not affect the OD₂₈₀. Rivanol did not precipitate any substances.

### EXAMPLE 7

The amount of precipitating agent required for manufacturing-scale use can be determined based on a 10,000 L growth chamber. Table 8 lists the proposed (1) concentrations and amounts of ethanol, zinc chloride, and PEG required for precipitation, (2) additional volume added by the precipitant, (3) hazards associated with the precipitant, (4) removal issues required for further downstream processing, and (5) special disposal requirements of the precipitant.

**TABLE 8**

| Precipitant Method | Approximate Concentrations | Total amount needed | Additional volume from precipitant | Hazards | Removal Issues | Special Disposal Required |
|---|---|---|---|---|---|---|
| Ethanol | 35% | 3,500 L | 3,500 L | Flammable | Possible | Yes |
| Zinc Chloride | 50 mM-80 mM | 5 Kg | 80 L | None | No | Yes |
| PEG 6000 | 6%-21% | 600-2100Kg | 3,000-20,000L | None | No | Yes |

### EXAMPLE 8

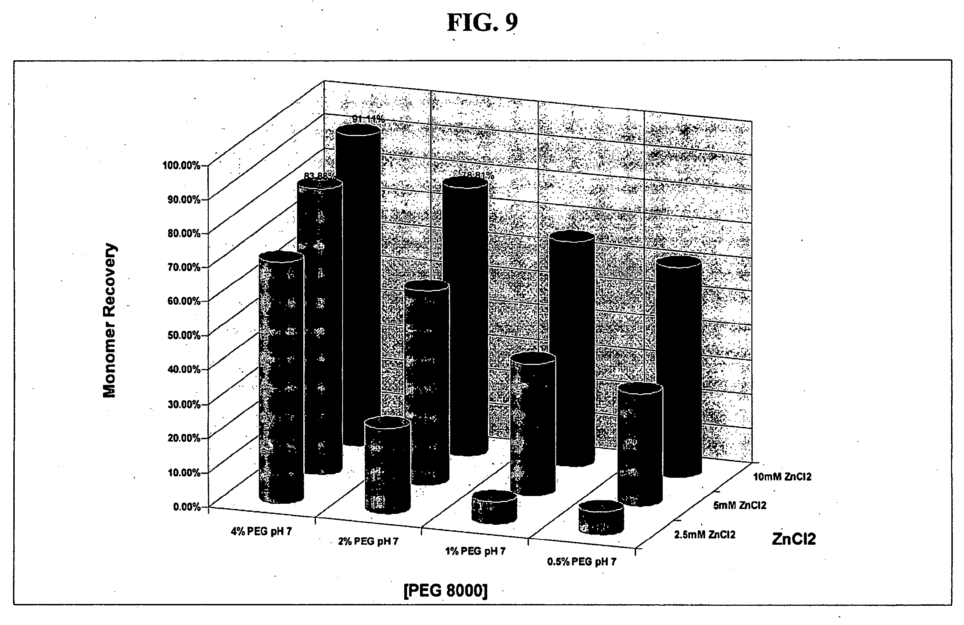

The affects of utilizing more than one precipitating agent was investigated on compositions containing the Ab C antibody at a pH of 7.0. The compositions were adjusted to contain either 0.5% PEG, 1% PEG, 2% PEG, or 4% PEG, and either 2.5 mM ZnCl₂, 5 mM ZnCl₂, or 10 mM ZnCl₂. The results are shown in FIG. 9.

As can be seen, the addition of both PEG and ZnCl₂ provided a significantly greater precipitating effect than just the PEG or the ZnCl₂ alone. The increased precipitating effect was also greater than the additive effect of the PEG or the ZnCl₂. Thus PEG and ZnCl₂ exhibit a synergistic effect on product recovery when used in concert.

### EXAMPLE 9

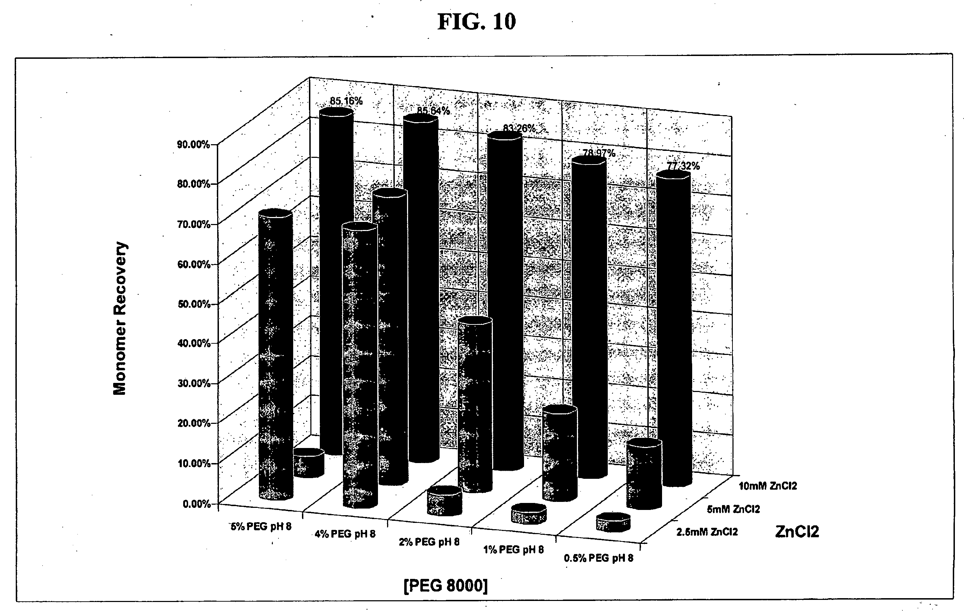

The affects of utilizing more than one precipitating agent was investigated on compositions containing the antibody Ab C at a pH of 8.0. The compositions were adjusted to contain either 0.5% PEG, 1% PEG, 2% PEG, or 4% PEG, and either 2.5 mM ZnCl₂, 5 mM ZnCl₂, or 10 mM ZnCl₂. The results are shown in FIG. 10.

As can be seen, the addition of both PEG and ZnCl₂ provided a significantly greater precipitating effect than just the PEG or the ZnCl₂ alone. The increased precipitating effect was also greater than the additive effect of the PEG or the ZnCl₂. Thus PEG and ZnCl₂ exhibit a synergistic effect on product recovery when used in concert.

### EXAMPLE 10

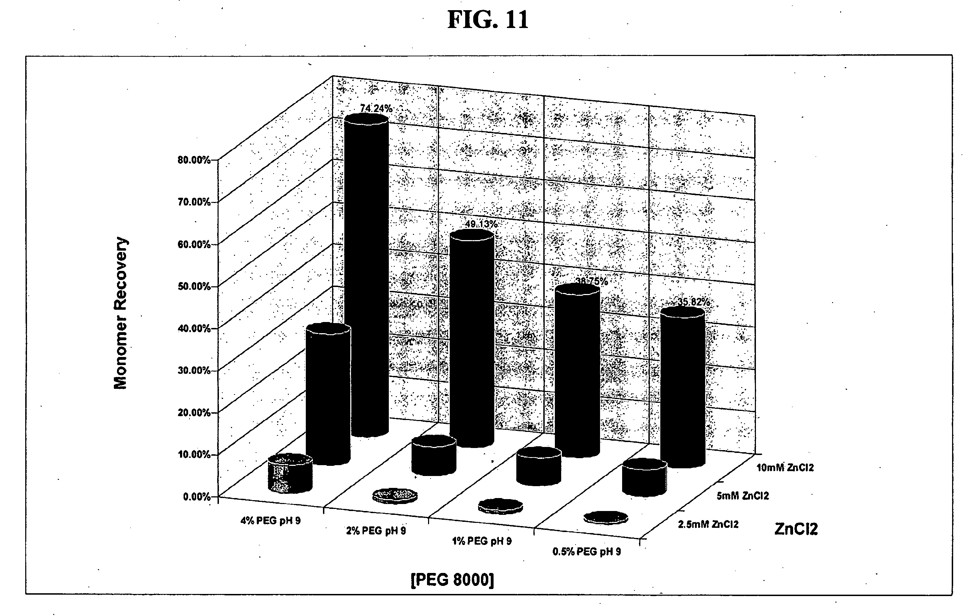

The affects of utilizing more than one precipitating agent was investigated on compositions containing the antibody Ab C at a pH of 9.0. The compositions were adjusted to contain either 0.5% PEG, 1% PEG, 2% PEG, or 4% PEG, and either 2.5 mM ZnCl₂, 5 mM ZnCl₂, or 10 mM ZnCl₂. The results are shown in FIG. 11.

As can be seen, the addition of both PEG and ZnCl₂ provided a significantly greater precipitating effect than just the PEG or the ZnCl₂ alone. This synergistic precipitating effect was also greater than the expected additive effect of PEG and ZnCl₂ together.

### EXAMPLE 11

The effect of PEG, ZnCl₂, and combinations of both, on the precipitation of various other antibodies was investigated. Four different antibodies were tested: two IgG1 antibodies, and two IgG4 antibodies. The results for the product recovery are presented in Table 9.

**TABLE 9**

| | **IgG1** | | **IgG4** | |
|---|---|---|---|---|
| | **Ab C** | **Ab D** | **Ab E** | **Ab F** |
| **5% PEG 3350** | 15% | 2% | 4% | 19% |
| **10 mM ZnCl₂** | 62% | 67% | 35% | 65% |
| **5% PEG 3350 + 10 mM ZnCl₂** | 91.1% | 93% | 90.1% | 92% |

The data in Table 9 demonstrates that the combination of PEG and ZnCl₂ provides more than an additive effect on the precipitation of various types and classes of antibodies. For example, the expected additive effect of adding 5% PEG (4%) and 10 mM ZnCl₂ (35%) to a composition comprising antibody Ab C would be 39%. However, a synergistic effect is observed because 91.1% of the antibody Ab C is precipitated.

### EXAMPLE 12

Precipitation of Ab F following concentration was performed by capture with Nutsche filtration and precipitate wash.

Harvested cell culture fluid (HCCF) of Ab F (initial concentration of 6.7 g Ab F/L of HCCF) was adjusted to pH 7.2 with 2M Tris Base at 2-8°C. The HCCF was concentrated using PELLICON-2® 50 kilo Dalton (kDa) cartridge (Millipore, Inc.) to 67 g/L, and the sample was then diafiltered (buffer exchanged) into 5mM Sodium Phosphate buffer at pH 7.2. (Note: concentration can be achieved using a variety of microfiltration/ultrafiltration membranes and filters or methods, for example using other concentration methods such as centrifugal buffer exchange or stirred cell). While vigorously stirring, a single bolus of a stock solution containing 250 mM zinc chloride at pH 3.0, was added to the concentrated sample to a final concentration of 2.5 mM. Following zinc chloride addition, a single bolus of 30% PEG 3350 and 100 mM imidazole was immediately added to the stirred sample, to a final concentration of 1.5% PEG 3350 and 10 mM imidazole. The resulting precipitate was stirred for 30 minutes. Diatomaceous earth (CELPURE® 1000) was then added to the stirred precipitate to a final concentration of 60 g/L (w/v) and fed to a dead-end Nutsche (pressurized) filter at constant flow rate. A precipitate cake was formed when the filter pressure reached 30 psi. The filter cake was subsequently washed with at least 3 volume equivalents of a series of solutions, including water. The resulting precipitate cake was then resolubilized with 40 mM EDTA, 60 mM Acetate at pH 5.0. Note: precipitate samples can resolubilized with a range of solutions, including 100 mM EDTA pH 7.2; 100 mM Acetate pH 5.0; 2.5 mM EDTA and 60 mM Acetate pH 5.0; 250 mM Tris pH 8.0. The resolubilized sample was then assayed for purity by size exclusion chromatography (SEC), SDS-PAGE and gel-chip SDS-PAGE, host cell protein and DNA content.

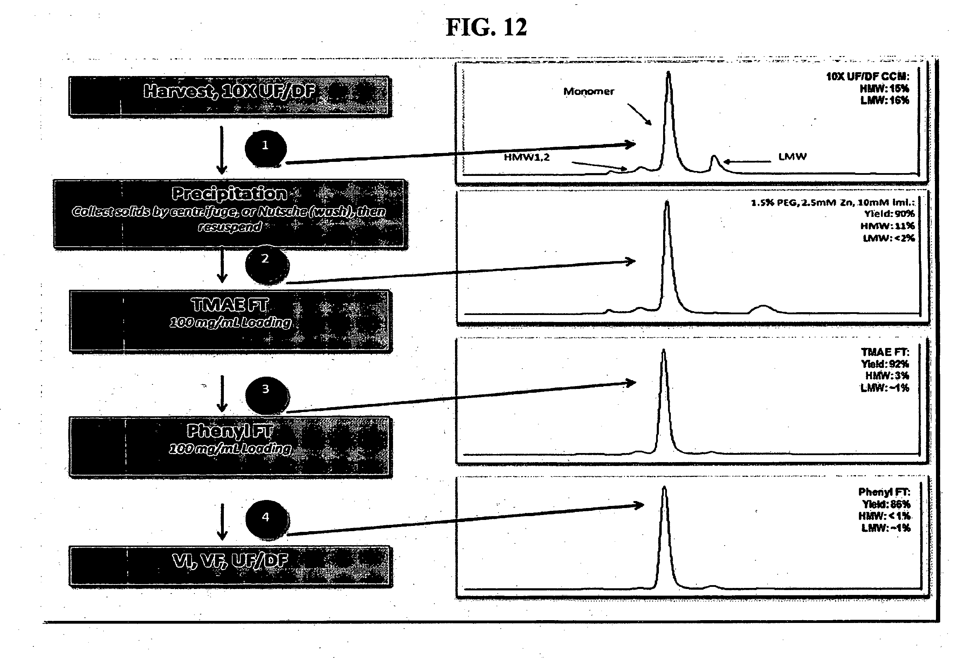

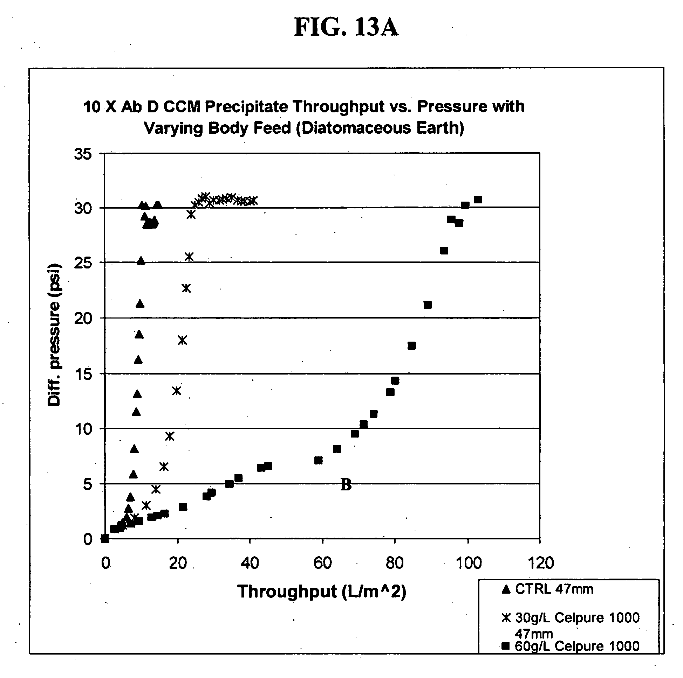

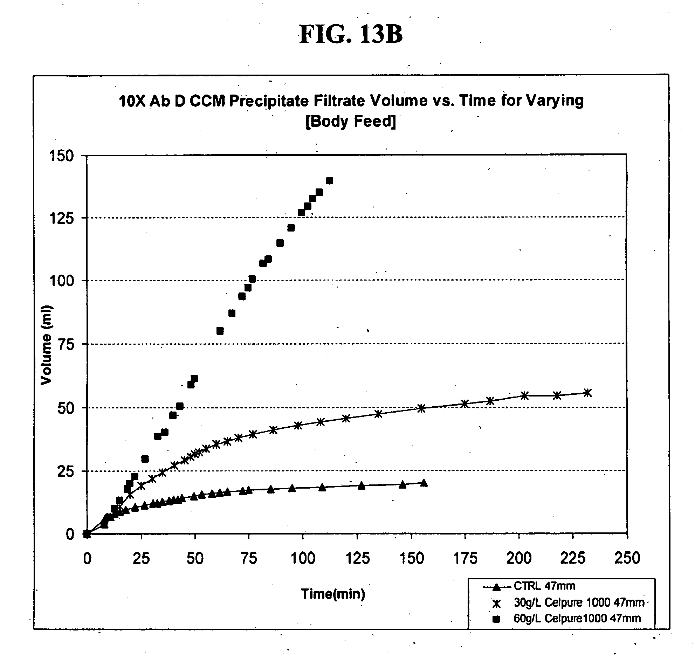

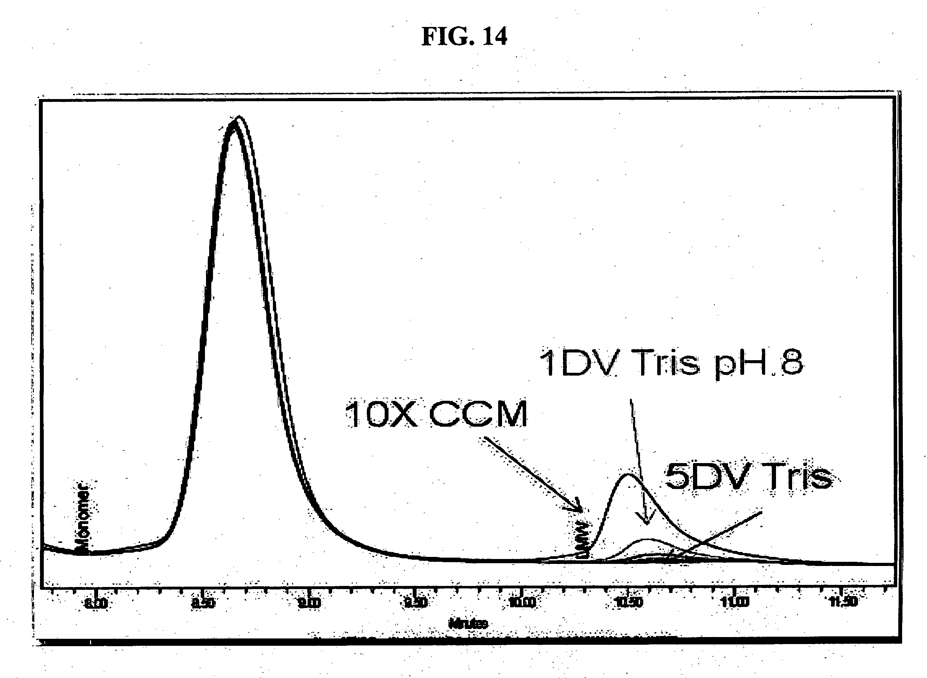

Resolubilized mAb was subsequently purified through two chromatographic steps: anion exchange chromatography, and hydrophobic exchange chromatography. Each resulting eluate was assayed by SEC, host cell protein and DNA content. Results are depicted in Figures 12-14.

### EXAMPLE 13

Precipitation of Ab F following concentration was carried out by capture and wash, and by settling and siphoning.

Harvested cell culture fluid (HCCF) of Ab F (initial concentration of 6.7 g Ab F/L of HCCF) was adjusted to pH 7.2 with 2M Tris Base at 2-8°C. The HCCF was concentrated using PELLICON-2® 50 kilo Dalton (kDa) cartridge (Millipore, Inc.) to 67 g/L, and the sample was then diafiltered (buffer exchanged) into 5mM Sodium Phosphate buffer at pH 7.2. (Note: concentration can be achieved using a variety of microfiltration/ultrafiltration membranes and filters or methods, for example using other concentration methods such as centrifugal buffer exchange or stirred cell). 35 mM zinc chloride was mixed with the concentrated sample to a final concentration of 2.5 mM. Following zinc chloride addition, 11% PEG 3350 and 200 mM imidazole were immediately added to the sample, to a final concentration of 1.5% PEG 3350 and 10 mM imidazole. The resulting precipitate was stirred for 30 minutes. The precipitate was allowed to settle for 2 hours and the supernatant was removed by suction. The precipitate cake was washed, by resuspending precipitate to original volume, with at least 2 volume equivalents of several solutions, including water, allowing the precipitate to settle for 2 h between each precipitate wash. The resulting precipitate cake was then resolubilized with 40 mM EDTA, 60 mM Acetate at pH 5.0. The resolubilized sample was then assayed for purity by size exclusion chromatography (SEC), SDS-PAGE and gel-chip SDS-PAGE, host cell protein and DNA content. Results are presented in Table 10.

**Table 10:**

| **Solution** | **Wash 1 Loss** | **Wash 2 Loss** | **Wash 3 Loss** | **LMW% in Resuspension** |
|---|---|---|---|---|
| Supernatant Control | 3.6% | - | - | 6.9% |
| Water | 1.3 % | 0.9 % | 0.5 % | 2.4 % |
| 2.5 mM ZnCl₂ | 0.5 % | 0.4 % | 0.4 % | 2.4 % |
| 1.25 mM ZnCl₂ | 0.8 % | 0.4 % | 0.3 % | 2.7 % |
| 0.63 mM ZnCl₂ | 1.0 % | 0.5 % | 0.3 % | 2.6 % |
| 1.5% PEG | 0.9 % | 0.5 % | 0.2 % | 2.6 % |
| 0.75% PEG | 1.2 % | 0.6 % | 0.4 % | 2.6 % |
| 0.25% PEG | 1.4 % | 0.8 % | 0.5 % | 2.7 % |

### EXAMPLE 14

Precipitation of Ab F with PEG 3350 and ZnCl₂ was carried out for the removal of low molecular weight impurities.

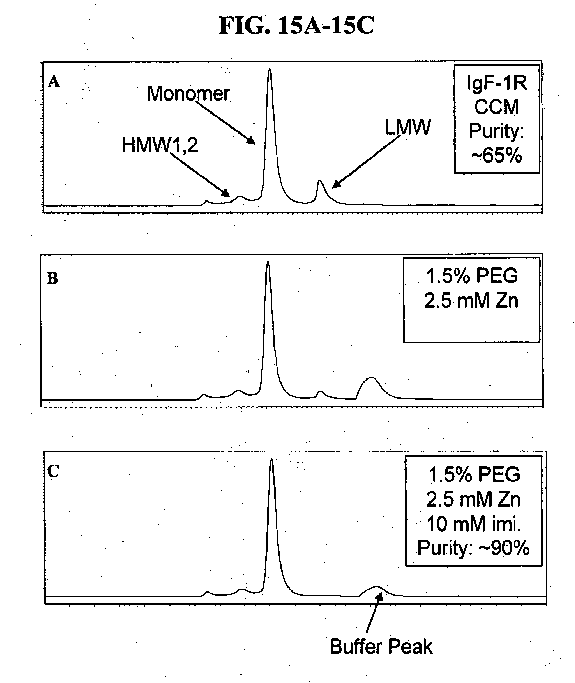

Harvested cell culture fluid (HCCF) of Ab F (initial concentration of 6.7 g Ab F/L of HCCF) was adjusted to pH 7.2 with 2M Tris Base at 2-8°C. The HCCF was concentrated using PELLICON-2® 50 kilo Dalton (kDa) cartridge (Millipore, Inc.) to 67 g/L, and the sample was then diafiltered (buffer exchanged) into 5mM Sodium Phosphate buffer at pH 7.2. (Note: concentration can be achieved using a variety of ultrafiltration membranes, including 50 kDa MWCO PELLICON-2®, or by other methods including centrifugal buffer exchange or stirred cell). While vigorously stirring, a single bolus of a stock solution containing 250 mM zinc chloride at pH 3.0, was added to the concentrated sample to a final concentration of 2.5 mM. Following zinc chloride addition, a single bolus of 30% PEG 3350 and 100 mM imidazole was immediately added to the stirred sample, to a final concentration of 1.5% PEG 3350 and 10 mM imidazole. The resulting precipitate was stirred for 30 minutes, sampled and resolubilized, and assayed by SEC to determine low molecular weight impurity content (LMW). Precipitation with ZnCl₂ and PEG 3350 in the presence of imidazole was found to reduce LMW impurity levels to nearly zero; precipitation with PEG and ZnCl₂ in the absence of imidazole was found to reduce levels of low molecular weight impurities from ∼16% to ∼6%. Results are displayed in Figure 15.

## Claims

1. A method of isolating an antibody in a composition containing an impurity, the method comprising:
(a) adding a polyalkylene glycol to the composition;
(b) adding a transition metal to the composition;
(c) allowing said antibody to form a precipitate in the presence of said polyalkylene glycol and transition metal; and
(d) separating said precipitated antibody from said impurity,
wherein step (a) and step (b) can be carried out in any order.

2. The method of claim 1, where the polyalkylene glycol is selected from the group consisting ofpolypentylene glycol, polybutylene glycol, polypropylene glycol, and polyethylene glycol.

3. The method of claim 2, where the polyalkylene glycol is polyethylene glycol.

4. The method of any one of claims 1-3, wherein the polyalkylene glycol is a polyethylene glycol which has a molecular weight selected from the group consisting of:
a) a molecular weight of between 1,000 Da and 20,000 Da; and
b) a molecular weight of between 2,000 Da and 15,000 Da.

5. The method of any one of claims 1-4, wherein the adding of the polyalkylene glycol results in a composition having a polyalkylene glycol concentration selected from the group consisting of:
a) a concentration of about 0.5% to about 30% (w/v); and
b) a concentration of about 2.0% to about 10% (w/v).

6. The method of any one of claims 1-5, wherein the transition metal is selected from the group consisting of zinc, nickel, copper, cobalt, and manganese.

7. The method of claim 6, wherein the transition metal is zinc.

8. The method of any one of claims 1-7, wherein the adding of the transition metal results in a composition having a transition metal concentration selected from the group consisting of:
a) a concentration of about 1 mM to about 50 mM; and
b) a concentration of about 2.5 mM to about 15 mM.

9. A method of any one of claims 1-8, wherein the separating of (d) is performed by:
a) filtering said composition, the filtering forming a permeate stream and a solid stream; or
b) subjecting said composition to centrifugation, said centrifugation forming a supernatant and a pellet.

10. The method of claim 9, wherein the separating of (d) is performed by filtering said composition, and wherein said filtering is performed by a dead-end filter.

11. The method of any one of claims 1-10, wherein said composition comprises eukaryotic cellular material.

12. The method of any one of claims 1-11, wherein said impurity is selected from the group consisting of a protein, lipid, nucleic acid, ribonucleic acid, growth media, and combinations thereof.

13. The method of any one of claims 1-11, wherein said impurity comprises a protein, nucleic acid, or combination thereof, and wherein said impurity originates from an organism that produced or contained said antibody.

14. The method of any one of claims 1-13, wherein the addition of said polyalkylene glycol and said transition metal increases the recovery of said antibody by an amount that is selected from the group consisting of:
a) greater than 3%; and
b) greater than 10%.

15. The method of any one of claims 1-14, wherein the separating of (d) is performed by filtering said composition, wherein the filtering results in a transmembrane pressure, and wherein said transmembrane pressure remains substantially constant during said filtering.

16. The method of any one of claims 1-15, wherein the polyalkylene glycol and the transition metal are the only precipitating agents.

17. The method of any one of claims 1-16, wherein a precipitate of the antibody is formed and then washed.

18. The method of claim 17, wherein the precipitate is washed with:
a) water;
b) a solution comprising ZnCl₂ at a concentration of about 0.5 mM to about 5 mM; or
c) a solution comprising a polyalkylene glycol at a concentration of about 0.1% to about 2.5%.

19. The method of any one of claims 1-18, further including use of a compound selected from the group consisting of:
a) imidazole;
b) glycine;
c) tryptophan;
d) cysteine;
e) histidine;
f) histamine; and
g) ammonium chloride
for isolation of the antibody.

20. The method of any one of claims 1-19, wherein a polyalkylene glycol is used for isolation of the antibody at a concentration (w/v) selected from the group consisting of:
a) a concentration in the range of about 0.5 % to about 5%;
b) a concentration of about 0.5 %;
c) a concentration of about 1 %;
d) a concentration of about 1.5 %;
e) a concentration of about 2 %;
f) a concentration of about 2.5 %;
g) a concentration of about 3 %;
h) a concentration of about 4 %; and
i) a concentration of about 5 %.

21. The method of any one of claims 1-19 wherein a transition metal is used for isolation of the antibody at a concentration selected from the group consisting of:
a) a concentration in the range of about 0.5 mM to about 5 mM;
b) a concentration of about 2.5 mM; and
c) a concentration of about 5 mM.

22. The method of any one of claims 1-19, wherein the polyalkylene glycol is added to the composition to result in a concentration of about 1.5 % (w/v) polyalkylene glycol and wherein the transition metal is added to the composition to result in a concentration of about 2.5mM transition metal.

23. The method of any one of claims 1-22, wherein the antibody is concentrated prior to addition of said polyalkylene glycol or transition metal.

24. The method of any one of claims 1-22, wherein the antibody is concentrated prior to addition of said polyalkylene glycol and transition metal.

25. The method of claim 23 or 24, wherein the prior concentration reduces the quantity of polyalkylene glycol or transition metal required for isolation of the antibody by an amount that is selected from the group consisting of:
a) about 2 to about 40-fold;
b) about 2-fold;
c) about 3-fold;
d) about 4-fold;
e) about 5-fold;
f) about 10-fold;
g) about 15-fold;
h) about 20-fold;
i) about 25-fold;
j) about 30-fold;
k) about 35-fold; and
l) about 40-fold.

26. The method of any one of claims 1 to 25, wherein the antibody retains biological activity, compared to the specific activity of the antibody prior to precipitation or prior to precipitation and washing, in an amount that is selected from the group consisting of:
a) about 50 % to about 100 % of biological activity;
b) about 50 % of biological activity;
c) about 60 % of biological activity;
d) about 70 % of biological activity;
e) about 80 % of biological activity;
f) about 90 % of biological activity;
g) about 95 % of biological activity; and
h) about 100 % of biological activity.

27. The method of any one of claims 1-26, wherein the steps (a), (b), (c) and (d) are carried out in the order of (a)-(b)-(c)-(d).

28. The method of any one of claims 1-26, wherein the steps (a), (b), (c) and (d) are carried out in the order of (b)-(a)-( c)-(d).

## Patentansprüche

1. Verfahren zur Isolierung eines Antikörpers in einer Zusammensetzung, die eine Verunreinigung enthält, wobei das Verfahren umfasst:
(a) Zugeben eines Polyalkylenglykols zu der Zusammensetzung;
(b) Zugeben eines Übergangsmetalls zu der Zusammensetzung;
(c) Ermöglichen, dass der Antikörper ein Präzipitat in der Gegenwart des Polyalkylenglykols und des Übergangsmetalls bildet; und
(d) Abtrennen des präzipitierten Antikörpers von der Verunreinigung, wobei Schritt (a) und Schritt (b) in beliebiger Reihenfolge durchgeführt werden können.

2. Verfahren nach Anspruch 1, wobei das Polyalkylenglykol ausgewählt ist aus der Gruppe bestehend aus Polypentylennglykol Polybutylenglykol, Polypropylenglykol und Polyethylenglykol.

3. Verfahren nach Anspruch 2, wobei das Polyalkylenglykol Polyethylenglykol ist.

4. Verfahren nach einem der Ansprüche 1-3, wobei das Polyalkylenglykol ein Polyethylenglykol ist, das ein Molekulargewicht ausweist, das ausgewählt ist aus der Gruppe bestehend aus:
a) einem Molekulargewicht zwischen 1.000 Da und 20.000 Da; und
b) einem Molekulargewicht zwischen 2.000 Da und 15.000 Da.

5. Verfahren nach einem der Ansprüche 1-4, wobei das Zugeben des Polyalkylenglykols in einer Zusammensetzung resultiert, die eine Polyalkylenglykolkonzentration aufweist, die ausgewählt ist aus der Gruppe bestehend aus:
a) einer Konzentration von etwa 0,5 % bis etwa 30 % (Gew./Vol.); und
b) einer Konzentration von etwa 2,0 % bis etwa 10 % (Gew./Vol.).

6. Verfahren nach einem der Ansprüche 1-5, wobei das Übergangsmetall ausgewählt ist aus der Gruppe bestehend aus Zink, Nickel, Kupfer, Kobalt und Mangan.

7. Verfahren nach Anspruch 6, wobei das Übergangsmetall Zink ist.

8. Verfahren nach einem der Ansprüche 1-7, wobei das Zugeben des Übergangsmetalls in einer Zusammensetzung resultiert, die eine Übergangsmetallkonzentration aufweist, die ausgewählt ist aus der Gruppe bestehend aus:
a) einer Konzentration von etwa 1 mM bis etwa 50 mM; und
b) einer Konzentration von etwa 2,5 mM bis etwa 15 mM.

9. Verfahren nach einem der Ansprüche 1-8, wobei das Abtrennen bei (d) durchgeführt wird durch:
a) Filtrieren der Zusammensetzung, wobei das Filtrieren einen Permeatstrom und einen Feststoffstrom bildet; oder
b) Unterziehen der Zusammensetzung einer Zentrifugation, wobei die Zentrifugation einen Überstand und ein Pellet bildet.

10. Verfahren nach Anspruch 9, wobei das Abtrennen bei (d) durch Filtrieren der Zusammensetzung durchgeführt wird und wobei das Filtrieren mit einem Dead-End-Filter durchgeführt wird.

11. Verfahren nach einem der Ansprüche 1-10, wobei die Zusammensetzung eukaryotisches Zellmaterial umfasst.

12. Verfahren nach einem der Ansprüche 1-11, wobei die Verunreinigung ausgewählt ist aus der Gruppe bestehend aus einem Protein, einem Lipid, einer Nukleinsäure, einer Ribonukleinsäure, einem Wachstumsmedium und Kombinationen davon.

13. Verfahren nach einem der Ansprüche 1-11, wobei die Verunreinigung ein Protein, eine Nukleinsäure oder eine Kombination davon umfasst, und wobei die Verunreinigung aus einem Organismus stammt, der den Antikörper produzierte oder enthielt.

14. Verfahren nach einem der Ansprüche 1-13, wobei die Zugabe des Polyalkylenglykols und des Übergangsmetalls die Gewinnung des Antikörpers um eine Menge erhöht, der ausgewählt ist aus der Gruppe bestehend aus:
a) mehr als 3 %; und
b) mehr als 10 %.

15. Verfahren nach einem der Ansprüche 1-14, wobei das Abtrennen bei (d) durch Filtrieren der Zusammensetzung durchgeführt wird, wobei das Filtrieren in einem Transmembrandruck resultiert, und wobei der Transmembrandruck während des Filtrieren im Wesentlichen konstant bleibt.

16. Verfahren nach einem der Ansprüche 1-15, wobei das Polyalkylenglykol und das Übergangsmetall die einzigen Präzipitationsmittel sind.

17. Verfahren nach einem der Ansprüche 1-16, wobei ein Präzipitat des Antikörpers gebildet und dann gewaschen wird.

18. Verfahren nach Anspruch 17, wobei das Präzipitat gewaschen wird mit:
a) Wasser;
b) einer Lösung, die ZnCl₂ in einer Konzentration von etwa 0,5 mM bis etwa 5 mM umfasst; oder
c) einer Lösung, die ein Polyalkylenglykol in einer Konzentration von etwa 0,1 % bis etwa 2,5 % umfasst.

19. Verfahren nach einem der Ansprüche 1-18, weiter einschließend Verwendung einer Verbindung ausgewählt aus der Gruppe bestehend aus
a) Imidazol;
b) Glycin;
c) Tryptophan;
d) Cystein;
e) Histidin;
f) Histamin; und
g) Ammoniumchlorid
zur Isolierung des Antikörpers.

20. Verfahren nach einem der Ansprüche 1-19, wobei ein Polyalkylenglykol zur Isolierung des Antikörpers in einer Konzentration (Gew./Vol.) verwendet wird, die ausgewählt ist, aus der Gruppe bestehend aus:
a) einer Konzentration im Bereich von etwa 0,5 % bis etwa 5 %;
b) einer Konzentration von etwa 0,5 %;
c) einer Konzentration von etwa 1 %;
d) einer Konzentration von etwa 1,5 %;
e) einer Konzentration von etwa 2 %;
f) einer Konzentration von etwa 2,5 %;
g) einer Konzentration von etwa 3 %;
h) einer Konzentration von etwa 4 %; und
i) einer Konzentration von etwa 5 %.

21. Verfahren nach einem der Ansprüche 1-19, wobei ein Übergangsmetall zur Isolierung des Antikörpers in einer Konzentration verwendet wird, die ausgewählt ist aus der Gruppe bestehend aus:
a) einer Konzentration im Bereich von etwa 0,5 mM bis etwa 5 mM;
b) einer Konzentration von etwa 2,5 mM; und
c) einer Konzentration von etwa 5 mM.

22. Verfahren nach einem der Ansprüche 1-19, wobei das Polyalkylenglykol zu der Zusammensetzung gegeben wird, um in einer Konzentration von etwa 1,5 % (Gew./Vol.) Polyalkylenglykol zu resultieren und wobei das Übergangsmetall zu der Zusammensetzung gegeben wird, um in einer Konzentration von etwa 2,5 mM Übergangsmetall zu resultieren.

23. Verfahren nach einem der Ansprüche 1-22, wobei der Antikörper vor der Zugabe des Polyalkylenglykols oder des Übergangsmetalls konzentriert wird.

24. Verfahren nach einem der Ansprüche 1-22, wobei der Antikörper vor der Zugabe des Polyalkylenglykols und des Übergangsmetalls konzentriert wird.

25. Verfahren nach Anspruch 23 oder 24, wobei die vorherige Konzentration die Menge an Polyalkylenglykol oder Übergangsmetall, die für die Isolierung des Antikörpers erforderlich ist, um eine Menge reduziert, die ausgewählt ist aus der Gruppe bestehend aus:
a) einer etwa 2 bis etwa 40-fachen;
b) einer etwa 2-fachen;
c) einer etwa 3-fachen;
d) einer etwa 4-fachen;
e) einer etwa 5-fachen;
f) einer etwa 10-fachen;
g) einer etwa 15-fachen;
h) einer etwa 20-fachen;
i) einer etwa 25-fachen;
j) einer etwa 30-fachen;
k) einer etwa 35-fachen; und
l) einer etwa 40-fachen.

26. Verfahren nach einem der Ansprüche 1 bis 25, wobei der Antikörper biologische Aktivität verglichen mit der spezifischen Aktivität des Antikörpers vor der Präzipitation oder vor der Präzipitation und dem Waschen in einer Menge beibehält, die ausgewählt ist aus der Gruppe bestehend aus:
a) etwa 50 % bis etwa 100 % biologischer Aktivität;
b) etwa 50 % biologischer Aktivität;
c) etwa 60 % biologischer Aktivität;
d) etwa 70 % biologischer Aktivität;
e) etwa 80 % biologischer Aktivität;
f) etwa 90 % biologischer Aktivität;
g) etwa 95 % biologischer Aktivität; und
h) etwa 100 % biologischer Aktivität.

27. Verfahren nach einem der Ansprüche 1-26, wobei die Schritte (a), (b), (c) und (d) in der Reihenfolge (a) - (b) - (c)-(d) ausgeführt werden.

28. Verfahren nach einem der Ansprüche 1-26, wobei die Schritte (a), (b), (c) und (d) in der Reihenfolge (b) - (a) - (c)-(d) ausgeführt werden.

## Revendications

1. Procédé d'isolement d'un anticorps dans une composition contenant une impureté, le procédé comprenant :
(a) l'ajout d'un polyalkylène glycol à la composition ;
(b) l'ajout d'un métal de transition à la composition ;
(c) le fait de permettre audit anticorps de former un précipité en présence desdits polyalkylène glycol et métal de transition ; et
(d) la séparation dudit anticorps précipité de ladite impureté,
dans lequel l'étape (a) et l'étape (b) peuvent être réalisées dans n'importe quel ordre.

2. Procédé selon la revendication 1, où le polyalkylène glycol est choisi dans le groupe constitué par le polypentylène glycol, le polybutylène glycol, le polypropylène glycol et le polyéthylène glycol.

3. Procédé selon la revendication 2, où le polyalkylène glycol est du polyéthylène glycol.

4. Procédé selon l'une quelconque des revendications 1 à 3, dans lequel le polyalkylène glycol est un polyéthylène glycol qui a un poids moléculaire choisi dans le groupe constitué par :
a) un poids moléculaire entre 1 000 Da et 20 000 Da ; et
b) un poids moléculaire entre 2 000 Da et 15 000 Da.

5. Procédé selon l'une quelconque des revendications 1 à 4, dans lequel l'ajout de polyalkylène glycol aboutit à une composition ayant une concentration en polyalkylène glycol choisie dans le groupe constitué par :
a) une concentration d'environ 0,5 % à environ 30 % (en poids/volume) ; et
b) une concentration d'environ 2,0 % à environ 10 % (en poids/volume).

6. Procédé selon l'une quelconque des revendications 1 à 5, dans lequel le métal de transition est choisi dans le groupe constitué par le zinc, le nickel, le cuivre, le cobalt et le manganèse.

7. Procédé selon la revendication 6, dans lequel le métal de transition est le zinc.

8. Procédé selon l'une quelconque des revendications 1 à 7, dans lequel l'ajout du métal de transition aboutit à une composition qui a une concentration en métal de transition choisie dans le groupe constitué par :
a) une concentration d'environ 1 mM à environ 50 mM ; et
b) une concentration d'environ 2,5 mM à environ 15 mM.

9. Procédé selon l'une quelconque des revendications 1 à 8, dans lequel la séparation de (d) est réalisée par :
a) la filtration de ladite composition, la filtration formant un flux de filtrat et un flux de solide ; ou
b) le passage de ladite composition à la centrifugation, ladite centrifugation formant un surnageant et un culot.

10. Procédé selon la revendication 9, dans lequel la séparation de (d) est réalisée par la filtration de ladite composition, et dans lequel ladite filtration est réalisée par un dispositif de filtration frontale.

11. Procédé selon l'une quelconque des revendications 1 à 10, dans lequel ladite composition comprend un matériau cellulaire eucaryote.

12. Procédé selon l'une quelconque des revendications 1 à 11, dans lequel ladite impureté est choisie dans le groupe constitué par une protéine, un lipide, un acide nucléique, un acide ribonucléique, un milieu de croissance, et les combinaisons de ceux-ci.

13. Procédé selon l'une quelconque des revendications 1 à 11, dans lequel ladite impureté comprend une protéine, un acide nucléique, ou une combinaison de ceux-ci, et dans lequel ladite impureté provient d'un organisme qui a produit ou a contenu ledit anticorps.

14. Procédé selon l'une quelconque des revendications 1 à 13, dans lequel l'ajout dudit polyalkylène glycol et dudit métal de transition augmente la récupération dudit anticorps dans une proportion qui est choisie à partir du groupe constitué par :
a) supérieur à 3 % ; et
b) supérieur à 10 %.

15. Procédé selon l'une quelconque des revendications 1 à 14, dans lequel la séparation de (d) est réalisée par la filtration de ladite composition, dans lequel la filtration aboutit à une pression transmembranaire, et dans lequel ladite pression transmembranaire reste sensiblement constante pendant ladite filtration.

16. Procédé selon l'une quelconque des revendications 1 à 15, dans lequel le polyalkylène glycol et le métal de transition sont les seuls agents de précipitation.

17. Procédé selon l'une quelconque des revendications 1 à 16, dans lequel un précipité de l'anticorps est formé et est ensuite lavé.

18. Procédé selon la revendication 17, dans lequel le précipité est lavé avec :
a) de l'eau ;
b) une solution comprenant ZnCl₂ à une concentration d'environ 0,5 mM à environ 5 mM ; ou
c) une solution comprenant un polyalkylène glycol à une concentration d'environ 0,1 % à environ 2,5 %.

19. Procédé selon l'une quelconque des revendications 1 à 18, incluant en outre l'utilisation d'un composé choisi dans le groupe constitué par :
a) un imidazole ;
b) la glycine ;
c) le tryptophane ;
d) la cystéine ;
e) l'histidine ;
f) l'histamine ; et
g) le chlorure d'ammonium
pour l'isolement de l'anticorps.

20. Procédé selon l'une quelconque des revendications 1 à 19, dans lequel un polyalkylène glycol est utilisé pour l'isolement de l'anticorps à une concentration (en poids/volume) choisie dans le groupe constitué par :
a) une concentration dans la plage d'environ 0,5 % à environ 5 % ;
b) une concentration d'environ 0,5 % ;
c) une concentration d'environ 1 % ;
d) une concentration d'environ 1,5 % ;
e) une concentration d'environ 2 % ;
f) une concentration d'environ 2,5 % ;
g) une concentration d'environ 3 % ;
h) une concentration d'environ 4 % ; et
i) une concentration d'environ 5 %.

21. Procédé selon l'une quelconque des revendications 1 à 19, dans lequel un métal de transition est utilisé pour l'isolement de l'anticorps à une concentration choisie dans le groupe constitué par :
a) une concentration dans la plage d'environ 0,5 mM à environ 5 mM ;
b) une concentration d'environ 2,5 mM ; et
c) une concentration d'environ 5 mM.

22. Procédé selon l'une quelconque des revendications 1 à 19, dans lequel le polyalkylène glycol est ajouté à la composition pour aboutir à une concentration d'environ 1,5 % (en poids/volume) de polyalkylène glycol et dans lequel le métal de transition est ajouté à la composition pour aboutir à une concentration d'environ 2,5 mM en métal de transition.

23. Procédé selon l'une quelconque des revendications 1 à 22, dans lequel l'anticorps est concentré avant l'ajout desdits polyalkylène glycol ou métal de transition.

24. Procédé selon l'une quelconque des revendications 1 à 22, dans lequel l'anticorps est concentré avant l'ajout desdits polyalkylène glycol et métal de transition.

25. Procédé selon les revendications 23 ou 24, dans lequel la concentration antérieure réduit la quantité de polyalkylène glycol ou de métal de transition nécessaire à l'isolement de l'anticorps d'une quantité qui est choisie dans le groupe constitué par :
a) environ 2 à environ 40 fois ;
b) environ 2 fois ;
c) environ 3 fois ;
d) environ 4 fois ;
e) environ 5 fois ;
f) environ 10 fois ;
g) environ 15 fois ;
h) environ 20 fois ;
i) environ 25 fois ;
j) environ 30 fois ;
k) environ 35 fois ; et
l) environ 40 fois.

26. Procédé selon l'une quelconque des revendications 1 à 25, dans lequel l'anticorps retient l'activité biologique, par comparaison avec l'activité spécifique de l'anticorps antérieure à la précipitation ou antérieure à la précipitation et au lavage, dans une quantité qui est choisie dans le groupe constitué par :
a) environ 50 % à environ 100 % de l'activité biologique ;
b) environ 50 % de l'activité biologique ;
c) environ 60 % de l'activité biologique ;
d) environ 70 % de l'activité biologique ;
e) environ 80 % de l'activité biologique ;
f) environ 90 % de l'activité biologique ;
g) environ 95 % de l'activité biologique ; et
h) environ 100 % de l'activité biologique.

27. Procédé selon l'une quelconque des revendications 1 à 26, dans lequel les étapes (a), (b), (c) et (d) sont réalisées selon l'ordre (a) - (b) - (c) - (d).

28. Procédé selon l'une quelconque des revendications 1 à 26, dans lequel les étapes (a), (b), (c) et (d) sont réalisées selon l'ordre (b) - (a) - (c) - (d).
